# EUROPEAN PATENT SPECIFICATION

(11) **EP 1 531 800 B1**
(45) Date of publication and mention of the grant of the patent: **08.06.2011**
(21) Application number: 03757174.2
(22) Date of filing: 11.06.2003
(51) Int. Cl.: A61K 9/51

(54) **STEALTH LIPID NANOCAPSULES, METHODS FOR THE PREPARATION THEREOF AND USE THEREOF AS A CARRIER FOR ACTIVE PRINCIPLE(S)**
STEALTH LIPID-NANOKAPSELN, VERFAHREN ZU IHRER HERSTELLUNG UND IHRE VERWENDUNG ALS TRÄGER VON WIRKSTOFF(EN)
NANOCAPSULES LIPIDIQUES INDETECTABLES, PROCEDES DE PREPARATION DE CELLES-CI ET UTILISATION DE CES NANOCAPSULES EN TANT QUE SUPPORT DE PRINCIPE(S) ACTIF(S)

(30) Priority: 11.06.2002 FR 0207175; 09.09.2002 US 421112 P
(43) Date of publication of application: 25.05.2005
(73) Proprietor: ETHYPHARM, 78550 Houdan (FR)
(72) Inventor: HOARAU, Didier, Montreal, Quebec H2T 2X3 (CA); DELMAS, Pascal, Quebec H2Y 3L8 (CA); LEROUX, Jean-Christophe, Montreal, Quebec H2Y 3Z2 (CA)
(74) Representative: Ahner, Francis
(86) International application number: PCT/IB2003/003213
(87) International publication number: WO 2003/103822

(56) References cited:
- WO-A-01/64328
- WO-A-98/51284
- WO-A-99/30620

## Description

The subject of the present invention relates to nanocapsules which, as a carrier for active principle(s), can be used for the treatment of solid or circulating cancerous tumors, which exhibit reduced toxicity compared to the free drug in solution, which exhibit stealth properties with respect to the immune system of the host to which they are administered and which are capable not only of undergoing extravasation into the tumor, but also of releasing their content therein. This novel carrier in accordance with the invention is advantageously in the form of an injectable colloidal suspension of lipid nanocapsules carrying at their surface phospholipid molecules associated with poly(ethylene glycol) molecules.

The present invention also relates to the method for synthesizing these stealth lipid nanocapsules, and in particular the method for inserting said molecules carrying poly(ethylene glycol) groups into the lipid envelope of the preformed nanocapsules. Finally, this invention relates at once to the uses which can be made of such a carrier, for delivering active principles via the blood, in particular for the treatment of solid or circulating tumors in humans, but also for the treatment of inflammations or of any other pathological condition for which an increase in vascular permeability is observed (inflammatory areas, infectious sites).

The present invention also relates to the various pharmaceutical compositions comprising the nanocapsules according to the present invention.

First of all, the term "carrier" is intended to mean any chemical entity which allows the transport and delivery, in the organism to which it is administered, of molecules, and in particular of active principles, such as medicinal products for example.

Moreover, the term "colloidal" is intended to mean any stable dispersion of solid elements with a size of less than 1 micrometer in a liquid phase.

The term "stealth" used to describe the carrier in accordance with the present invention denotes the ability of the nanocapsules not to be detected and then sequestered and/or degraded, or to be hardly detected and then sequestered and/or degraded, and/or to be detected and then sequestered and/or degraded late, by the immune system of the host to which they are administered.

The term "extravasation" is used to denote the exit of a body from the blood compartment. This exit takes place through pores or "fenestrations", tangentially to the axis of the blood capillary. This phenomenon is only observed in certain organs, the capillaries of which are fenestrated, such as the kidney or the liver, for example, and in particular extracerebral solid tumors and inflamed or infected tissues whose blood vessel exhibit increased permeability.

The term "nanocapsules" is intended to mean spheres with a diameter of less than 300 nm comprising a circular wall more or less rigid at ambient temperature and a central component or core which, in the case of the present invention, contains fatty substances.

The term "amphiphilic" is used to describe a molecule carrying groups having an affinity for substances which are hydrophobic in nature and groups having an affinity for substances which are hydrophilic in nature.

Finally, "mol%" expresses a molar percentage and the term "active principle" denotes a pharmaceutically active principle.

The stealth lipid nanocapsules which are the subject of the present invention consist of an essentially lipid core which is liquid or semi-liquid at ambient temperature, and an outer lipid envelope comprising at least one hydrophilic surfactant which is lipid in nature, at least one lipophilic surfactant which is lipid in nature and at least one amphiphilic derivative of poly(ethylene glycol) (PEG), the molar mass of the poly(ethylene glycol) component of which is greater than or equal to 1 000 g/mol, preferably greater than or equal to 2 000 g/mol.

The "pegylated" amphiphilic derivative contributes to conferring the stealth aspect on the nanocapsules. The core of the nanocapsules advantageously allows the incorporation and transport of many molecules, and more particularly of active principles which are lipophilic in nature, such as anticancer agents, transported in dissolved or dispersed form. The nanocapsules in accordance with the invention are thus capable of carrying anticancer medicinal products in the blood system, and more particularly in the interstitial medium bordering tumors, for sustained periods of time. Moreover, the nanocapsules in accordance with the invention have a sufficiently long blood circulation time, allowing them to undergo interactions with circulating tumor cells and greater extravasation in solid tumors.

"Solid" tumors consist of tumor cells organized in solid clumps or in tissues irrigated by the blood circulation. Many tumors are irrigated by a network of fenestrated capillaries. These capillaries thus have types of pores which, depending on their size, allow solid elements to pass out of the blood compartment. The size of the fenestrations between the endothelial cells of the basal membrane of tumor vessels varies from one tumor to another. However, colloidal particles less than 300 nm in size can undergo extravasation in some tumors. This permeating of colloidal particles to the interstitial medium of the tumor is, moreover, combined with an increased retention effect due to poor lymphatic drainage of the tumor. This double phenomenon, named EPR for *Enhanced Permeability and Retention,* has made it possible to envisage the development of an antitumor therapy based on colloidal carrier systems. Specifically, EPR allows significantly sustained accumulation, compared to that which is observed on healthy tissues, of colloidal particles and of macromolecules in the area around the tumor.

This EPR phenomenon has thus been exploited in order to form colloidal carriers intended to transport anticancer active principles. However, in order to be truly effective, such carriers must be able to circulate in the blood compartment for a sufficient amount of time before being detected and then destroyed by the host's immune system. Thus, non-stealth colloidal objects are rapidly detected by certain effectors of the immune system, in particular such as macrophages, and cannot reach tumors in sufficient amounts to be effective.

In fact, macrophages constitute one of the most important components of the immune system and play a predominant role in eliminating foreign particles from the blood circulation, including liposomes and other colloidal particles.

The recognized expression for denoting this set of circulating and sedentary monocytes which constitute the non-specific defence system involved in capturing nanoparticles is "the Mononuclear Phagocyte System" (MPS).

At the molecular level, the clearance of injected particles takes place in two steps: opsonization by the depositing of serum proteins (or "opsonins") at the surface of the particles followed by recognition and capture of the opsonized particles by macrophages.

Modification of the surface of nanoparticles with chains of hydrophilic and flexible polymers, generally of the poly(ethylene glycol) type, confers them a steric protection by preventing the opsonins reaching the surface of the particles and bringing about the elimination thereof by the cells of the MPS. The effectiveness of these polymer chains in conferring a stealth nature depends both on their length and on their density. Thus, Mori et al. (FEBS Lett., 284: 263, 1991) have carried out comparative studies with phospholipids comprising chains of PEG 750, 2 000 and 5 000 g/mol, present at the surface of 200 nm liposomes. At equal molar percentage, PEG 750 proved to be completely ineffective *in vitro* in conferring steric protection against opsonization. On the other hand, the longest PEG chains exhibit an effectiveness against opsonization which is related to their length. This *in vitro* behaviour is correlated with the half-lives of the liposomes, after intravenous injection into mice, which are: less than 30 minutes, approximately 2 hours and more than 3 hours for PEG 750, PEG 2000 and PEG 5000, respectively.

Other studies (Allen et al., BBA, 1066: 29, 1991) demonstrated that, below 1 000 g/mol, the poly(ethylene glycol) chains, even when they are at saturating concentration at the surface of the nanoparticles, do not allow them to confer a sufficient steric protection and therefore stealth properties.

The design of stealth carriers intended to transport anticancer active principles makes it possible not only to increase their therapeutic effect, but also to thus ensure a certain invisibility with respect to the opsonization phenomenon and the immune system. Moreover, this increase in their blood half-life time (consequence of the stealth effect) may also serve to decrease the toxicity of the carried active principles, which will mainly be released in the interstitial tissue of the tumor with very limited diffusion in healthy tissues.

In fact, anticancer active principles are generally molecules which are small in size and which exhibit a high cytotoxicity, particularly pronounced on dividing cells. Moreover, the effective therapeutic concentration thereof is sometimes not very different from the plasma concentration at which they become toxic for the organism to which they are administered. This narrow concentration window requires the use of carriers for the administration of such compounds. The use of stealth carriers allows them to accumulate in the interstitial tissue of tumors and to release their active agents therein. Due to this accumulation, the intratumoral concentration of active principle is very high, much greater than the concentration which can be reached using conventional carriers or with the active principle alone (Allen, Trends, Pharm. Sci., 15: 215, 1994). Stealth colloidal systems therefore make it possible to use higher doses of anticancer agents, greater than those which it is possible to administer with the active principles alone due to their high toxicity.

The accumulation of nanoparticles in tumors and the resulting therapeutic effect depends on the half-lives of their elimination from the blood circulation. Thus, various formulations of liposomes of the conventional type or exhibiting sustained circulation have been compared on the basis of their blood elimination profile and of their accumulation in tumors in mice (Gabizon A. and Papahadjopoulos D., Proc. Natl. Acad. Sci. USA, 85: 6949, 1988). Thus, a stealth formulation having a half-life of approximately 8 hours shows an accumulation of more than 10% of the injected dose in the tumors after 24 hours. Control formulations having half-lives of slightly less than one hour show accumulations in tumors which are much lower than that observed for the stealth formulation. Specifically, the intratumoral concentrations of the control formulations, at 24 h, represent only 4 to 16% of the amount assayed in the tumors in the case of the stealth formulation. These results show that formulations having half-lives of the order of one hour or less than an hour accumulate poorly in tumors.

Colloidal systems such as liposomes or nanoparticles, i.e. consisting of particles in suspension which are 25 nm to 1 µm in diameter, led to the development and the commercialisation of various colloidal pharmaceutical forms.

Thus, US patent No. 5,811,119 describes the use of liposomes for administration of anticancer medicinal products derived from carotenoids. This liposomal formulation in particular makes it possible to decrease the *in vivo* toxicity of these active principles. The carotenoids intended to treat tumors are here combined with an intercalation-promoting agent which allows their loading in the liposome. The transport of these lipophilic active principles in a liposome, the core of which is hydrophilic in nature, is possible due to the presence of amphiphilic compounds such as triglycerides. These "intercalation-promoting agents" make it possible to transport carotenoids in a ratio of 10 mol of triglycerides to one mole of carotenoid.

Such a carrier makes it possible to envisage transporting anticancer agents while decreasing their cytotoxicity, but has a low encapsulation rate and, moreover, allows neither a precise targeting of the tumors nor an increase in the lifetime of these carriers in the blood compartment. Specifically, the major drawback of the liposome-type system is its poor stability in the circulation due, in particular, to its rapid biodegradation by circulating enzymes.

Document No. US 5,527,528 describes the use of modified liposomes for carrying and administering anticancer medicinal products. The liposomes described are between 50 and 120 nm in size, contain an antitumor compound such as cytarabine and have, at their surface, molecular chains of poly(ethylene glycol) at a sufficient concentration to significantly increase the circulation time of said liposomes in the blood compartment of the host to which they are administered. Moreover, these liposomes also carry, at their surface, antibodies specific for certain tumor antigens, allowing active targeting of the liposomes against the tumors.

That document discloses a carrier which provides both sustained transport of active principles in the circulation and a certain targeting to the type of tumor against which they are directed.

However, this type of carrier has a major drawback. Specifically, it is especially advantageous for anticancer agents which are hydrophilic in nature, such as cytarabine or gentamycin for example. Here again, the transport of lipophilic active principles, and in particular of anticancer agents such as daunomycin for example, is limited. These compounds are transported by incorporation into the double phospholipid layer of the liposomes. Because of this, the incorporation yield remains low since the volume of the core of the liposome is not used for the transport. The main drawback of these systems is represented by a limited capacity of incorporation for lipophilic medicinal products due to the unfavourable ratio of the aqueous compartment volumes to the lipophilic compartment volumes.

In addition, the membrane incorporation of active principles will depend on their degree of solubilization in the lipid environment of this phospholipid double layer. It is difficult to modulate the lipophilic nature within this bilayer given that the choice of the components thereof, in this case the phospholipids, is limited. This type of carrier does not therefore make it possible to adjust the HLB* (Hydrophilic Lipophilic Balance) of the transport medium with regard to the liposolubility of the active principle transported with as much ease as the core of the nanocapsules in accordance with the invention for which the choice of the constituents of the core offers greater versatility. The incorporation yields are therefore dependent on the degree of solubility of the active principle in the liposomal membrane. Thus, the liposomes are generally incapable of incorporating more than 5 mol% (most commonly 1 to 2%) of hydrophobic active principles into their membrane relative to all the lipids making up the composition of the lipid bilayer (Lasic, Trends Biotechnol. 16: 307, 1998).

*The HLB index, or hydrophilic/lipophilic balance, is as defined by C. Larpent in treatise K.342 of the publications *"Techniques de l'ingénieur".*

The liposomes sold under the trade mark Doxil^{®} and intended to transport doxorubicine are also objects which exhibit certain stealth properties in the blood compartment. These liposomes have, at their surface, molecules of PEG inserted into the phospholipid double layer, and thus provide passive targeting with respect to the tumors.

This active principle is "loaded" into the liposomes by a mechanism of *"active loading"* which results from the complexation of doxorubicin as it diffuses into the aqueous core. The pH gradient allows the doxorubicin to enter the liposomes, where this active principle will then change into the form of a gel. This filling mechanism allows a high active principle loading yield to be obtained.

However, this mechanism can only be exploited for certain active principles which are amphiphilic and ionisable in nature, which can readily diffuse across the lipid bilayer (therefore small molecules) and which have precipitation or complexing properties, such as doxorubicin or mitoxantrone, and is not applicable for most anticancer agents. The active principles which cannot benefit from this mechanism exhibit low loading yields and require subsequent steps of separation on a column and of recycling of the active principle.

In addition, the use of liposomes as carriers for active principles has other drawbacks. In particular, the presence of the phospholipid double layer physically limits the size of the liposomes obtained, which may, with difficulty, reach sizes of less than approximately 50 nm due to the limiting value of the radius of curvature which may be attained by this membrane.

The use of liposomal carriers therefore implies certain major drawbacks, in particular in their ability to transport several anticancer substances, but also with regard to the ease with which they can be used and stored.

Specifically, such liposomal carriers cannot be easily lyophilized without losing their structural properties and their capacity to transport when the colloidal solution is reconstituted. As a result, liposomal suspensions may exhibit quite poor stability and relatively short conservation times.

In order to attempt to overcome these drawbacks which limit the effectiveness of this type of carrier, nonliposomal colloidal carriers have been developed. Polymer-based micelles in fact offer a good alternative to liposomes in terms of transport of lipophilic active principles, and in particular of anticancer agents.

Thus, document No. WO 02/00194 describes a colloidal carrier in the form of polymer-based micelles. This carrier allows, inter alia, the transport and delivery of anticancer active principles such as doxorubicin or methotrexate for example. The micelles claimed comprise a hydrophobic central component into which a lipophilic anticancer agent can be inserted, and an outer "shell" which is hydrophilic in nature. These micelles in fact correspond to an assembly of diblock polymers comprising a hydrophilic half based on poly(vinylpyrrolidone) and a hydrophobic half consisting of poly(lactic acid). The hydrophobic central component corresponds to the coming together of the hydrophobic central components of the polymer molecules making up these micelles. This carrier, besides its ability to transport hydrophobic molecules, makes it possible to provide particles which are very small in size, and in particular less than 100 nm, which is difficult to achieve in the case of liposomes.

Other types of polymeric micelles have also been developed with the aim of vehiculing active principles in the blood for sustained times (Stolnik et al., J. Drug Target., 9: 361, 2001). This type of carrier also has a structure of the diblock polymer type, the hydrophobic component consisting of poly(lactic acid) (PLA) and the hydrophilic components being represented by PEG. Depending on the length of each of the blocks, the PLA-PEG micelles obtained have various sizes, more generally around 75 nm. Intravenous injection of these micelles into rats reveals certain stealth properties with an elimination half-life of between 1 and 2 hours; 25% of the injected dose is still present in the blood after 3 hours.

On the other hand, the micelle-based carriers have the drawback that they are relatively unstable in the organism and can rapidly dissociate upon dilution. In fact, the stability of the micelles depends on the critical micelle concentration (CMC), below which the polymeric molecules which form them disassemble. This relative stability makes the industrial use of micelles for the therapeutic delivery of anticancer substances delicate.

Thus, carriers based on nanoparticles, other than liposomes, have been developed in order to overcome the lack of stability of the existing colloidal systems and/or their poor effectiveness in transporting molecules which are lipophilic in nature.

Thus, patent WO 01/64328 describes such lipid nanocapsules comprising a rigid lipid surface and a liquid or semi-liquid core composed of esters of fatty acids and of triglycerides. The rigid surface of these nanocapsules is composed, in part, of Lipoid^{®} S 75-3, which corresponds to a mixture of several lecithins, and of Solutol^{®} HS 15. This type of carrier makes it possible to envisage the transport of hydrophobic active principles, and in particular of anticancer agents. In such nanoparticles, the lipophilic active principle is present in dissolved form inside the core, the composition of which may be modified so as to adjust the HLB as a function of the liposolubility of the active principle chosen. The lipid nanocapsules according to patent WO 01/64328 have been the subject of an evaluation of their behaviour in the blood *in vivo,* subsequent to the intravenous injection thereof into rats (Cahouet et al., Int. J. Pharm. 242:367 2002). Solutol^{®} HS 15 which makes up the surface of these nanoparticles is a molecule consisting of a hydrophobic component and a short chain of poly(ethylene glycol) of 660 g/mol, protruding outside. As mentioned above, PEG chains which are so short, even at high density, are not sufficiently effective to confer on the nanoparticles a steric protection against opsonization and therefore stealth properties. Thus, the half-life time of these lipid nanocapsules is short since it is approximately 45 minutes. These lipid nanocapsules circulate, however, for a longer period of time than small molecules, such as radioactive labels alone. The half-life time of the lipid nanocapsules in accordance with patent WO 01/64328 would not therefore be sufficient to allow them to circulate for sustained periods of time in order to be able to accumulate in tumors (in organs other than the liver and the spleen), as mentioned previously.

Other types of lipid nanocapsules exhibiting, this time, a certain long circulation time in mice, and intended to transport anticancer active principles, have been produced and described by Mosqueira et al. (Pharm. Res., 18: 1411, 2001). Such lipid nanocapsules have both a core composed of fatty substances which allows lipophilic active principles, in particular anticancer agents, to dissolve and therefore to be transported, and a rigid outer layer of polymers onto which PEG chains are grafted, providing them with some stealth properties with respect to the immune system.

These nanocapsules, approximately 200 nm in diameter, exhibit a blood circulation time which is significantly greater than that of the same nanoparticles lacking said surface molecules. However, these results were obtained in mice, the immune system of which has a capacity for opsonization which is less than that observed in rats or in humans for example. Their relative effectiveness in evading the immune system of mice does not therefore make it possible to extrapolate these results to the immune systems of rats or humans, which are more powerful in terms of capacity for opsonization (Liu D. et al., BBA, 1240: 277, 1995).

Moreover, their relatively large diameter would not allow them to readily undergo extravasation in most solid tumors. Thus, even though the time of residence in the blood compartment is increased, the tumor targeting remains low, which limits all the more their therapeutic effectiveness.

Nanospheres composed of polymers made up of a diblock structure of PLA-PEG, exhibiting stealth properties in rats, with an elimination half-life of 6 hours, have also been developed (Verrecchia et al., J. Control. Rel. 36: 49, 1995). The hydrophobic component of the polymer consists of PLA of 30 000 g/mol, and the hydrophilic chain is composed of PEG of 2 000 g/mol. These nanoparticles have a surface which is relatively unfavourable to complement activation, the consequence of which is to decrease opsonization phenomena and phagocytosis by the cells of the MPS.

However, the major drawback of this type of nanoparticle based on solid polymers of the PLA type lies in their poor capacity to release the active principle rapidly, once in the area around the clump of tumor cells. Specifically, the rigid outer layer of these nanocapsules is composed of PLA and is degraded relatively slowly by hydrolysis. This weak bioerosion implies a slow release of the active principles contained in these nanocapsules. Now, the therapeutic effectiveness of such anticancer carriers comes, firstly, from their ability to evade the immune system and, secondly, from their ability to rapidly release, i.e. in less than 24 hours, their content in the area around the tumor. Now, the nanoparticles described by Mosqueira or Verrecchia are practically undegraded after having been in the organism for a period of 24 hours, which does not make it possible to envisage an optimal treatment of solid tumors.

The present invention thus provides a novel carrier for active principle(s), which makes it possible both to avoid most of the drawbacks observed with regard to liposomal carriers, and to improve the effectiveness of targeting and of release of active principle(s) to/in the tumor compared to the existing nonliposomal colloidal carriers.

The aim of the present invention is also to provide a novel type of stealth carrier for active principle(s), which allows more effective treatment of cancers, in particular solid or circulating tumors by increasing their therapeutic index.

The therapeutic index is the ratio of the drug dose which produces an undesired effect to the dose which causes the desired effects.

Owing to their reduced toxicity, since the drug is encapsulated and not in solution, their long circulating properties and tumor targeting capabilities, the nanocapsules according to the present invention provide the drug with an improved therapeutic index. In fact, encapsulation of active principles in colloidal drug carriers has been shown to reduce their distribution volume. This alteration in the pharmacokinetic profile leads to increased efficacy and/or reduced toxicity and enables higher amounts of drug to be administered.

Specifically, the nanocapsules according to the present invention, as a carrier for active principle(s), may be used for the treatment of solid tumors since the size and furtiveness characteristics of the nanocapsules in accordance with the invention allow these nanocapsules to approach and penetrate these tumors via a phenomenon of extravasation. In this case, reference is made to passive targeting of the tumors. Thus, to the applicant's credit, it has been able to design a carrier composed of stealth lipid nanoparticles allowing passive targeting of the tumors at which it is aimed.

In addition, the nanocapsules according to the present invention, as a carrier for active principle(s), may be used for the treatment of circulating tumors via a phenomenon of active targeting. This active targeting is produced by attaching, to the surface of said nanocapsules, molecules or "ligands" specific for certain surface molecules of the circulating tumors. The attachment of such surface molecules is advantageously carried out during the method for synthesizing the nanocapsules, by post-insertion, as described later. In general, the nanocapsules in accordance with the invention, as a carrier for active principle(s), may be used for the treatment of tumors which are solid or circulating in nature, by active targeting, when they have receptors which can be recognized by a ligand, or by passive targeting (without the involvement of a receptor).

The nanocapsules according to the present invention, as a carrier for active principle(s), also exhibit rapid and therefore more effective release of this (these) active principle(s) into the immediate environment of the tumor. Such a rapid release is permitted by the rapid bioresorption of the constituents of said nanocapsules, in particular due to the action of the enzymes present in the organism.

This characteristic of biodegradation, in particular by enzymatic digestion, is an additional advantage of the present invention, which makes it possible to improve the therapeutic effectiveness of the active principles administered in this way. The carrier in accordance with the invention also exhibits advantageous stealth properties with respect to the immune system and complete biodegradability combined with a total lack of toxicity. In fact, all the constituents of the carrier in accordance with the invention are biodegradable and can be rapidly assimilated in the organism, are all the subject of an approval for parenteral use and are also formulated without any organic solvent. Thus, this "intrinsic" toxicity of the nanocapsules is very low or does not even exist.

Moreover, the nanocapsules according to the present invention are able to carry relatively high content of potentially toxic active principle (like anticancer agents) through the organism showing reduced toxicity compared with free drug in solution.

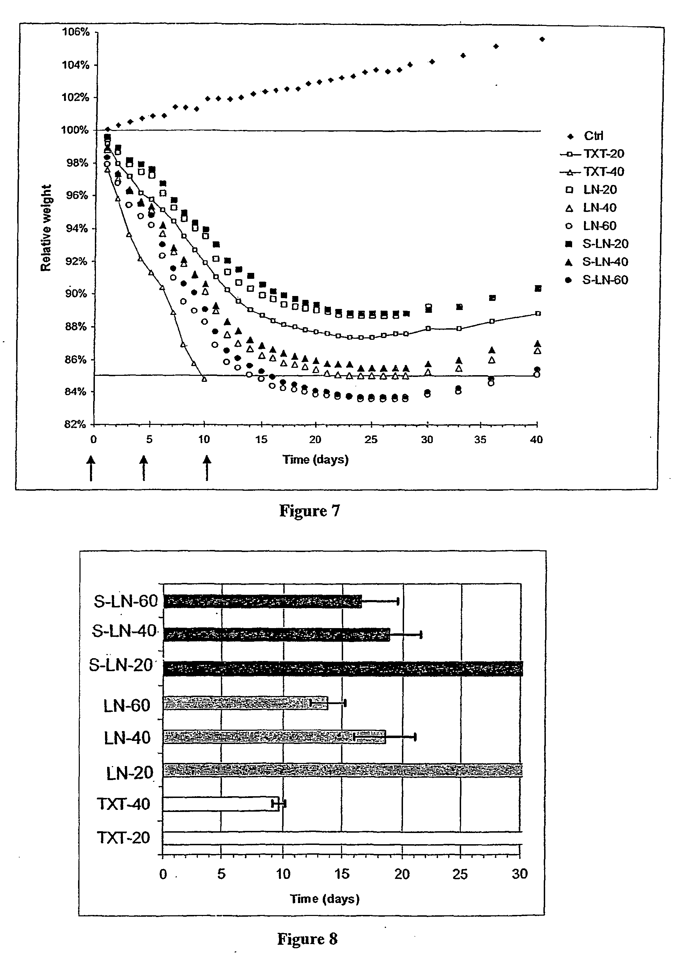

This aspect of the present invention is shown in example 10, part F) of the specification and on figure 7 (Evolution of the relative average body weight of mice after three intravenous injections of docetaxel) and figure 8 (average number of days to reach 15 % body weight loss for different formulations of docetaxel administered to mice).

Thus, their very low intrinsic toxicity, combined with their reduced toxicity when carrying potentially toxic substance, allow the nanocapsules according to the present invention to become a very safe carrier of anticancer agents in mammals.

The novel stealth colloidal carrier in accordance with the invention also has the advantage of being readily lyophilizable and sterilizable, without losing its properties when it is reconstituted in the form of a suspension.

This carrier also makes it possible to administer, via the vascular route, medicinal products intended for the treatment of tissue inflammation.

The lipid nanocapsules according to the present invention advantageously have a diameter of between 50 and 150 nm, preferentially of between 80 and 120 nm.

The size of said nanocapsules plays a role in the passive targeting of the tumoral anticancer agents. Specifically, the EPR effect observed in the tumor allows the passage into, and the retention in, the interstitial medium which surrounds the solid tumor of elements present in the blood, the diameter of which is sufficiently small to allow this extravasation. The nanocapsules in accordance with the invention are prepared using a method which advantageously makes it possible to modulate their size. Moreover, they exhibit long circulating properties which provide them with a half-life time in the blood compartment of greater than two hours. These two characteristics advantageously make it possible to promote the extravasation phenomenon by increasing the number of passages made by the nanocapsules in the region of the fenestrations of the tumor capillaries.

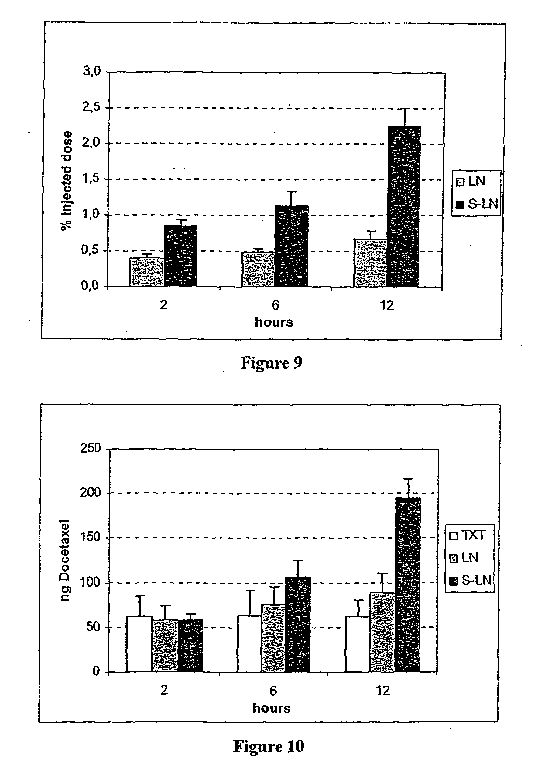

The extravasation phenomenon is shown in example 11, part G) (see also figures 9 and 10) where accumulation of docetaxel loaded radio-labelled nanocapsules in tumours is shown to be significantly more important for stealth nanocapsules (S-LN) than for non-pegylated nanocapsules (LN).

The stealth nature of the nanocapsules in accordance with the invention is also capable of promoting the contacts with malignant cells present in the blood circulation, in the case of circulating tumors.

Extravasation corresponds to a phenomenon of tangential filtration, which means that a tumor having pores of a diameter of 200 nm will allow circulating objects with the same diameter to pass only with difficulty. Moreover, the size of the nanocapsules in accordance with the invention allows them to avoid capture by extravasation at the level of the cells of the liver. In fact, the fenestrations of the sinusoidal capillaries of the liver allow the extravasation of particles of about 50 nm. Finally, said nanocapsules must be less than 200 nm in size in order to avoid the phenomenon of filtration observed in the spleen for particles greater than this in size. Thus, the defined size of the nanocapsules is responsible both for the phenomenon of passive targeting promoted by EPR, and for the increase in the amount of time said nanocapsules persist in the blood circulation by enabling them to avoid the phenomena of filtering and hepatic extravasation.

The diameter of the nanocapsules can be adjusted as a function of the type of tumor to be treated and may, where appropriate, be slightly less or more than the abovementioned range.

The size of the nanocapsules must, in fact, be adjusted to the size of the fenestrations of the tumors to be destroyed, this size possibly varying over time, depending on the maturity of the tumor, and in space, within the same tumor. As a result, the passive targeting of the carrier in accordance with the invention can only be envisaged on a therapeutic level if the size of the nanocapsules in accordance with the invention can be precisely adjusted. Now, the applicant has advantageously developed a method for precisely synthesizing stealth nanocapsules of variable and controllable size. The present carrier thus comprises abilities to adapt to the tumor environment which make it all the more effective.

The outer lipid envelope of the nanocapsules according to the present invention comprises a lipophilic surfactant which is lipidic in nature. This surfactant is advantageously in the form of a lecithin, the phosphatidylcholine proportion of which is at least equal to 95%, preferentially greater than 99%.

The applicant has noted that the constitution of nanocapsules comprising a phosphatidylcholine content of greater than 95% makes it possible, in rats, to significantly increase the circulation time in the blood circulation. Thus, impurities such as other phospholipids, for instance phosphatidylethanolamine, lysophospholipids or free fatty acids, may contribute to increasing the phenomenon of opsonization of these nanoparticles. In addition, the applicant has also noted that the yield of insertion of the molecules of lipid coupled to the molecules of PEG is better when the lipophilic surfactant exhibits an initial phosphatidylcholine concentration of greater than 95%.

Thus, the observed long circulation time of the nanocapsules according to the invention may come from the fact that the outer surface of these nanocapsules consists of a practically pure phospholipid based on phosphatidylcholine, which limits opsonization. The degree of purity in terms of phosphatidylcholine plays a role in the mechanisms for avoiding opsonization.

The lipophilic surfactant which is lipidic in nature advantageously represents a molar percentage of between 5 and 30 mol% of the lipid molecules making up said outer lipid envelope.

This surfactant preferably has a temperature of transition from gel to liquid phase of greater than 25°C, preferentially greater than 37°C; this means that, at ambient temperature, said surfactant is in gel form and only changes to liquid form at a temperature of greater than 25°C, preferentially greater than 37°C. The gel/liquid transition temperature plays a role in the rigidity of the outer lipid envelope of said nanocapsules. The rigidity of the outer envelope offers the nanocapsules according to the invention a considerable physicochemical stability, both in aqueous medium when they are in suspension, but also when they are administered in the blood compartment. In fact, the rigidity of said outer envelope confers on the nanocapsules a cohesion which allows them to withstand environmental modifications such as the variations in pH or in osmotic pressure which occur during the injection.

Advantageously, the lipophilic surfactant is a phospholipid carrying saturated carbon-based chains, preferably two chains, each of these chains comprising at least 16 carbon atoms. As a result of this, a phospholipid may preferably be chosen from C₁₈-C₁₈ DSPC (distearoylphosphatidylcholine), C₁₆-C₁₈ HSPC (hydrogenated soy phosphatidylcholine) and C₁₆-C₁₆ DPPC (dipalmitoylphosphatidylcholine). Preferentially, HSPC may be used at a degree of purity of greater than 99% so as to obtain better rigidity and better stealth properties. The latter compound is part of the composition of an injectable liposomal formulation sold under the trade mark Doxil^{®}.

Moreover, said outer lipid envelope comprises a hydrophilic surfactant which is lipid in nature, preferably approved for parenteral use. This hydrophilic surfactant is advantageously chosen from poly(ethylene glycol) alkyl esters and poly(ethylene glycol) alkyl ethers, and mixtures thereof.

This hydrophilic surfactant preferably represents between 60 and 90 mol% of the lipid molecules making up said outer lipid envelope. This surfactant is preferably used in a molar proportion of approximately 80 mol% of the lipid molecules making up the outer lipid envelope.

This hydrophilic surfactant is used, with the lipophilic surfactant, for the formation of the oil/water emulsion required for preparing the nanocapsules according to the invention. It in fact ensures stabilization of the fatty droplets formed from the mixture of triglycerides during the method for synthesizing said nanocapsules. Moreover, this surfactant also ensures the cohesion of said outer lipid envelope. The relatively high proportion of hydrophilic surfactant in the envelope of said nanocapsules makes it possible to obtain a low interfacial tension, making it possible to prepare nanocapsules in which the higher the proportion of hydrophilic surfactant, the smaller the diameter.

Use will preferably be made of the nonionic surfactants sold under the names: Solutol^{®} HS15 (BASF), Brij^{®} (Uniquema) or Mapeg^{®} (BASF). Use will preferentially be made of Solutol^{®} HS15, which corresponds to poly(ethylene glycol)-660 12-hydroxystearate, comprising a short chain of 15 units of ethylene glycol corresponding to a molecular weight of 660 g/mol. This surfactant is advantageously approved for parenteral use, and more particularly for intravenous administration; in addition, it gives good results in terms of cohesion.

Finally, said outer lipid envelope comprises an amphiphilic derivative of PEG which contributes effectively to the long circulation time of said nanocapsules. Specifically, the amphiphilic derivative of PEG makes it possible at once to decrease the phenomenon of opsonization of the nanocapsules, to improve the stability of the colloidal solution and to prolong the residence time for these nanocapsules in the blood circulation.

The amphiphilic derivative of PEG advantageously comprises a hydrophobic component which allows it to be anchored in said outer lipid envelope and a hydrophilic component of the PEG type facing the outside of said lipid nanocapsules, conferring hydrophilic properties at the surface thereof.

The amphiphilic derivatives of PEG are inserted into the outer envelope of said nanocapsules via their hydrophobic end. Consequently, the PEG component faces the outside of said nanocapsules and forms a protective hydrophilic shell surrounding each of the nanocapsules. The hydrophobic end allows anchoring at the surface of the outer envelope. This anchoring is provided by the low energy bonding forces which link the hydrophobic components of the pegylated lipids to those of the surfactants present at the surface of said lipid envelope. Specifically, the cohesion of the outer lipid envelope of the nanocapsules in accordance with the invention results from the Van der Waals forces which are exerted between the hydrophobic groups, and in particular the alkyl chains of the molecules making up this envelope. These low energy bonds make it possible at once to maintain sufficient cohesion between the components of the envelope to avoid too rapid a degradation of the nanocapsules in accordance with the invention when they are introduced into the blood compartment, but confer upon them, however, sufficient fragility to ensure rapid biodegradability and therefore a therapeutically effective release of the active principles transported.

In fact, unlike the polymeric chains of the PLA (poly lactic acid) or PLAGA (poly (lactic-co-glycolic acid)) type which constitute the surface of the nanocapsules as described by Mosqueira, and which are in solid form at any temperature under physiological conditions, the fatty chains which make up the envelope of the nanocapsules according to the invention are not covalently bound to one another, and are therefore more subject to disassembly. Specifically, bioerosion is a surface phenomenon which is the consequence of enzymatic attack or of the natural biodegradation (or bioresorption) linked to the local pH conditions and to the presence of water. This erosion is responsible for the loss of phospholipids at the surface of the nanocapsules which facilitate the exit of the active principles.

The polymer chains of the nanocapsules based on PLA or PLAGA are long (45 000 g/mol for the nanocapsules described by Mosqueira) and constitute a thick surface which is both rigid and smooth. The biodegradation thereof is therefore all the slower. Thus, more than 24 hours (or even several weeks) are necessary for the biodegradation of the nanocapsules based on PLA or on PLAGA to be complete. The phospholipids present at the surface of the nanocapsules according to the invention are distinct entities and advantageously allow extraction of the surface and rapid biodegradation, necessary for a therapeutically effective release of the active principles transported.

Finally, the constituents of the outer envelope of the nanocapsules are all not only biodegradable and approved for parenteral use and intravenous injection, but they can also be assimilated by the organism. Specifically, constituents such as phosphatidylcholine or phosphatidylethanolamine are commonly present in food and are assimilated by the organism naturally.

Thus, the size of the chains covering the surface of the nanocapsules, and also the nature thereof and the nature of the chemical bonds which keep them assembled, contribute to making the nanocapsules according to the invention both stable in the blood compartment for more than an hour and rapidly biodegradable and able to be assimilated by the circulating enzymes or enzymes present in the interstitial fluids.

Advantageously, the half-life of the nanocapsules in accordance with the invention is greater than 2 hours. Ideally, for the nanocapsules in accordance with the invention, the intention will be to attain a plasmatic half-life of between 3 and 10 hours. This characteristic advantageously allows the nanocapsules according to the invention to accumulate in the tumors and to liberate therein most of their contents within reasonable amounts of time. The effectiveness of the anticancer agent therefore increases as a result. Now, the rapidity of release of the antitumor agent is a primordial factor to be taken into account for eliminating solid tumors, given their high rate of growth.

The amphiphilic derivatives of PEG used in the present invention preferentially have, as a hydrophobic group, a phosphatidylethanolamine. They are therefore pegylated phospholipids. Thus, use may be made of biodegradable phospholipids, in particular chosen from
DPPE-PEGₓ (dipalmitoylphosphatidylethanolamine),
DSPE-PEGₓ (distearoylphosphatidylethanolamine),
DOPE-PEGₓ (dioleoylphosphatidylethanolamine), and
POPE-PEGₓ (palmitoyloleylphosphatidylethanolamine),
in which x represents the size of the PEG molecule in g/mol, and also mixtures thereof.

Preferentially, as a phospholipid coupled to a molecule of PEG, DSPE-PEGₓ will be used for its qualities of stability in the outer envelope of the nanocapsules in accordance with the invention.

Advantageously, DSPE-PEG₂₀₀₀, DSPE-PEG₃₀₀₀ and DSPE-PEG₅₀₀₀ will be used.

Moreover, the stealth effect of the nanocapsules according to the present invention may also be obtained with pegylated lipids of the sterol nucleus-PEG, ceramide-PEG or PEG₂₀₀₀ sorbitan tetraoleate type, or more generally of the alkyl-PEG type having at least one alkyl chain providing hydrophobic anchoring to at least one molecule of PEG in the outer envelope.

The size of the molecule of PEG polymer grafted to the phospholipid described above is also of importance. Specifically, it will condition the spatial hindrance at the surface of said nanocapsules and, consequently, the accessibility to this surface but also the ability of these nanocapsules to evade the MPS.

The molecules of PEG of the amphiphilic derivatives used in the present invention preferably have a molar mass of greater than or equal to 1 000 g/mol. As mentioned above, the presence of PEG chains less than 1 000 g/mol in size does not confer sufficient protection against opsonization. Consequently, the short chain of PEG of 660 g/mol present on the molecule of hydrophilic surfactant Solutol^{®} HS15 mentioned above could not confer effective protection of the surface against opsonization, and therefore sustained circulation. The molecules of PEG of formula (O-CH₂-CH₂)n which can be used in the context of the present invention belong to the group comprising PEG₅₀₀₀, comprising 113 PEG units, PEG₃₀₀₀, comprising 68 PEG units, and PEG₂₀₀₀, comprising 45 PEG units. These long pegylated chains confer a hydrophilic nature at the surface of the lipid nanocapsules.

The molar proportion of the amphiphilic derivative of PEG integrated into the envelope of said nanocapsules is preferably between 0.5 and 12 mol% of the lipid molecules making up the outer lipid envelope, preferably between 1 and 10 mol%. Preferentially, the molecules of DSPE-PEG will be used in a molar proportion of between 1 and 10 mol% of the total lipids of the envelope.

In a preferred embodiment, molecules of DSPE-PEG₅₀₀₀ will be used since their size (they are approximately twice as long as the molecules of DSPE-PEG₂₀₀₀) makes it possible, for the same number of molecules, to obtain greater repulsion via the steric effect and more significant stealth properties *in vivo.* Specifically, the PEG chains are in constant movement at the surface of the lipid envelope, and thus, the longer they are the more they sweep a large space around the lipid nanocapsules, which increases the steric hindrance and decreases all the more the possibility for proteins to reach the surface of these nanocapsules. The ability to evade the phenomenon of opsonization is therefore greater in the presence of DSPE-PEG₅₀₀₀.

The core of the nanocapsules in accordance with the invention is an essentially lipid core composed of fatty acid esters and/or of triglycerides and/or oil as fatty substance and/or mixtures thereof. The triglycerides are advantageously chosen from medium chain triglycerides carrying from 6 to 14 carbon atoms and/or caprylic/capric triglycerides, and also mixtures thereof. More preferentially, use will be made, as a mixture of fatty substances making up the core of the nanocapsules in accordance with the invention, of Labrafac^{®} cc, Miglyol^{®} 812 N or Miglyol^{®} 810 N which correspond to the following designations in the Pharmacopoeia: mixture of medium chain triglycerides, fractionated coconut oil or caprylic/capric triglycerides. The triglycerides may be composed of pure glyceryl tricaprylate ( 8 carbons in the alkyl chain), like Tricaprylin. The triglycerides may be used alone or as a mixture with other pharmaceutical oils or fatty acid esters which are also the subject of an approval for parenteral use, in order to maximize the amount of drug to be dissolved in the core.

The fatty acid esters making up said essentially lipid core are advantageously chosen from the medium chain fatty acids carrying from 8 to 18 carbon atoms, such as ethyl palmitate, ethyl oleate, ethyl myristate, isopropyl myristate or octyldodecyl myristate, and also mixtures thereof.

Of course, the composition and the proportions of each of these constituents can be modulated and adjusted as a function of the hydrophilic/lipophilic balance (or HLB) desired for the mixture. In fact, since the inner face of the envelope is lipophilic in nature, there will be no incompatibility between the nature of the envelope and the content of the core.

These lipids are in liquid or semi-liquid form at ambient temperature depending on the size and on the degree of saturation of the carbon-based chains carried by the triglycerides and the oils constituting said core. The presence of fatty substances inside the core of the nanocapsules according to the invention contributes to acting as a solvent for the anticancer agents transported, but also constitutes a solid mass which is used for maintaining and for the relative stability of these nanocapsules in the external environment, whether this is in solution in an aqueous liquid or in the dry state at the end of a lyophilization step. Unlike the liposomes or the other colloidal carriers which have a lipid membrane and an aqueous interior, the presence of a fatty substance content therefore provides the nanocapsules according to the invention with a certain stability (variation in ionic strength or in osmotic pressure).

Advantageously, the outer surface of the outer lipid envelope of the stealth lipid nanocapsules which are the subject of the present invention is hydrophilic in nature and the essentially lipid core is lipophilic in nature.

Thus, the combination of biodegradable lipids which can be assimilated, which make up the core and the envelope, both makes it possible to envisage a rapid release of the active principles transported, and also provides the nanocapsules with a physical stability which allows them to be lyophilized and even sterilized by passing them through a 0.45 to 0.22 µm filter. The nanocapsules in accordance with the invention may therefore be sterilized and, optionally, stored in dry form for long periods of time. The novel carrier in accordance with the invention thus offers a certain ease of use which makes it a product which is readily exploitable in industry.

Advantageously, the essentially lipid core of the stealth lipid nanocapsules which are subjects of the present invention represents between 20 and 60%, preferably between 25 and 50%, by weight relative to the total weight of said nanocapsules.

Advantageously, the aqueous phase or dispersant phase, in which the nanocapsules (or dispersed phase) in accordance with the invention may be prepared, initially contains a certain concentration of salts, advantageously of NaCl. This concentration is between 4 and 8%, advantageously within the range of 4.4 to 5.2%. At the end of the method of synthesis, osmotic agents, cryoprotective agents or lyoprotective agents and, optionally, preserving agents may be added to this aqueous phase.

The applicant has in fact noted that this concentration of salt makes it possible at once to obtain a phase inversion temperature (or TIP) which is lower than in the absence of salts, to allow a more massive incorporation of phospholipid molecules coupled to PEG in the case of the conventional method of preparation (described hereafter) and, finally, to significantly decrease the size of the nanocapsules obtained.

The fatty substance mixture constituting said core makes it possible to envisage carrying weakly to strongly lipophilic active principles, and more particularly anticancer substances. Specifically, depending on the liposolubility of each active principle, the HLB will be adjusted to allow complete dissolution of the active principle. The present invention even allows the transport of a mixture of active principles of different solubility, for which an intermediate HLB will be applied, ensuring at least partial solubility of each anticancer agent.

Advantageously, the stealth lipid nanocapsules which are subjects of the present invention contain one or more active principles, in particular which are lipophilic in nature.

The active principle(s) may be dissolved or dispersed in the lipid core of the nanocapsule.

Thus, the following anticancer active principles, which are mainly lipophilic in nature, may be used, alone or as a mixture: paclitaxel and derivatives thereof such as docetaxel, camptothecin and derivatives thereof such as irinotecan, topotecan or rubitecan, busulfan, chlorambucil, phthalocyanins, carotenoids and daunomycin in particular.

Moreover, anticancer active principles which are amphiphilic in nature, such as cytarabin, cyclophosphamide, methotrexate, fluoro derivatives such as 5-fluorouracil or 5-fluorouridine, for example, or doxorubicin in particular, may also be transported by the nanocapsules in accordance with the invention. The active principles will be dissolved beforehand in the fatty acid esters or the triglycerides or in pharmaceutical oils and fatty substances which are of injectable grade. Organic co-solvents, approved for parenteral administration, may be used in order to solubilize active principles which are poorly soluble in pharmaceutical oils; the solution obtained may then be mixed to these oils. The solutions of active principle thus prepared may be directly used in the method or may be the subject of subsequent dilutions in triglyceride mixtures, before they are used.

Finally, it is entirely possible to envisage the transport of any other type of active principle, and in particular of agents for treating inflammations or sites of infections in tissues. Thus, the nanocapsules in accordance with the invention, by virtue of their stealth properties and the fact that their size can be modulated, are particularly suitable for carrying and delivering medicinal products to tissues in which there is inflammation. Specifically, during inflammation, as observed in forms of arthritis for example, the fenestrations of the capillaries irrigating the inflamed area become enlarged so as to allow increased transport of certain elements present in the blood, in particular of proteins. The same is true of tissues in which there are sites of infection. As a result, even though the accumulation in these areas is less marked than in tumors, it is possible to envisage that the release of suitable active principles, in a region close to the inflammation or the infection, by nanocapsules in accordance with the invention makes it possible to reduce the consequences of such pathological conditions. Thus, it is entirely possible to envisage the transport, by the nanocapsules in accordance with the invention, of anti-inflammatory substances, such as prostaglandin E1, nonsteroidal anti-inflammatories such as ibuprofen, ketoprofen, indomethacin or corticoids such as dexamethasone and also antibiotics, analgesics and anti-infectious agents such as amphotericin B or ketoconazole.

The core of the nanocapsules in accordance with the invention is therefore composed of a fatty phase, the variable composition of which makes it possible to adjust the ability of the nanocapsules to solubilize a given anticancer agent.

Advantageously, the stealth lipid nanocapsules which are the subject of the invention are able to reduce the toxicity (and severity of adverse effects) of the carried active principle(s), allowing greater amounts of drug to be administrated and thus improving the therapeutic index of potent but highly toxic drugs. Moreover, owing to their long circulating properties and their size, the stealth lipid nanocapsules in accordance with the invention are able to accumulate and thus increase the amount of drug in tumor tissues compared to conventional solutions of anticancer agents. Tumor treatment's efficacy is expected to be considerably enhanced since more drug may be administrated (owing to reduced toxicity against healthy tissues) and more drug may be delivered to the tumor tissue (owing to the specific extravasation at the tumor site).

A further object of the present invention is the use of the nanocapsules of the present invention for the preparation of a medicament wherein the toxicity of the active principle(s) against the healthy tissues is reduced.

A subject of the present invention is also the pharmaceutical compositions comprising the nanocapsules according to the present invention, which contain one or more active principles. These pharmaceutical compositions are advantageously in the form of a colloidal aqueous suspension containing said nanocapsules.

Such a carrier may be administered parenterally, and in particularly intravenously, intra-arterially, intraperitonealy, intra-articularly and intramuscularly, but it is also possible to envisage injecting it subcutaneously. In fact, subcutaneous injection will make it possible to reach the lymphatic vessels which may ensure accumulation of the nanocapsules according to the invention in the lymph nodes. This route of access is therefore particularly important for the treatment of lymph node ailments, and more particularly lymph node tumors.

### 1) Treatment of solid and circulating tumors

The present invention can be used, firstly, in anticancer therapies via the blood.

The nanocapsules in accordance with the invention may be used to treat solid tumors in humans.

Moreover, they may be used to treat circulating tumors of the leukaemia or lymphoma type. In fact, as has been mentioned above, the targeting of circulating tumor cells can be obtained only if the particles of the colloidal system have a circulating time sufficient to promote encounters with the tumor cells before any capture by the liver or circulating MPS cells. Thus, the nanocapsules in accordance with the invention are particularly suitable for the treatment of circulating tumors.

These nanocapsules may also have capacities for active targeting by means of the coupling of ligands having specific affinity for molecules of the receptor type located at the surface of the cancer cells.

Advantageously, the stealth lipid nanocapsules which are subjects of the present invention carry, at their surface, specific ligands which confer upon them the ability to actively target cells having receptors for these ligands, in particular tumor cells.

Preferably, the ligand will be chosen so as not to stimulate, or to slightly stimulate, the phenomena of clearance of the particles.

Advantageously, said ligand is of the saccharide or oligosaccharide type or vitamin type, or alternatively of the oligopeptide, antibody fragment or monoclonal antibody type.

The ligands of the saccharide or oligosaccharide type or vitamin type (folate, riboflavin), and even of the oligopeptide, antibody fragment or monoclonal antibody type, may advantageously be used. Thus, said ligands will be coupled, beforehand, to the reactive end of molecules of the activated pegylated phospholipid type or any other lipid which may comprise a hydrophobic component, able to be anchored in the surface of the nanocapsules, a hydrophilic chain of the PEG type and a reactive chemical function at the end of this hydrophilic chain. These ligands, once attached to the end of these chains, will then preferentially be introduced using the post-insertion technique as described below. The post-insertion method in fact makes it possible to introduce a particular type of ligand or ligands which are different in nature having diverse specificities capable of conferring a set of particular properties on the carrier in accordance with the invention.

Therefore, a further object of the present invention is the use of the lipid nanocapsules of the present invention for the preparation of a medicament for the treatment of cancers, in particular solid or circulating tumors, by intravenous administration, or for the preparation of a medicament for treating circulating or solid tumors by active targeting, or for the preparation of a medicament for treating solid tumors by passive targeting subsequent to the extravasation of said nanocapsules through the tumor capillaries.

### 2) Treatment of inflamed tissues (in particular arthritis) and infected tissues

The use of the nanocapsules in accordance with the invention for the purpose of treating tissue infections and/or inflammations, in particular in humans, may also be envisaged. Specifically, as was described above, during tissue inflammations or infections, the capillaries irrigating the area subject to inflammation experience increased permeability. This temporary permeability can be exploited in the same way as for the treatment of solid tumors, using the phenomenon of extravasation to introduce into the inflamed tissue bioactive substances capable of decreasing or eliminating the causes and/or the consequences of the inflammation. Thus, it is possible to envisage, in particular, the transport, by the nanocapsules in accordance with the invention, of active principles intended to attenuate the inflammation, such as anti-inflammatories, analgesics or antibiotics for example. In the case of arthritis for example, which corresponds to inflammation of certain joints, an intra-articular injection of the nanocapsules in accordance with the invention will preferentially be given. This is because the targeting of the carrier directly to its site of action is thus promoted, and the loss by dilution of the nanocapsules in the entire blood circulation is, moreover, limited. Furthermore, the stealth properties of the nanocapsules in accordance with the invention should allow them not to be recognized and eliminated from the sites of inflammation by the large number of cells of the MPS and of the immune system present at these sites.

Therefore, a further object of the present invention is the use of the lipid nanocapsules according to the present invention for the preparation of a medicament for treating inflammations and/or infections of tissues.

### 3) Detoxification

Advantageously, the lipid nanocapsules which are subjects of the present invention may be used to take up hydrophobic molecules present in the blood circulation subsequent to an instance of poisoning.

After instances of acute poisoning with an active principle which is hydrophobic in nature, stealth lipid nanocapsules which have not been loaded with an active principle may be administered intravenously. Thus, by virtue of their sustained circulation time and of their hydrophobic lipid core, the nanocapsules in accordance with the invention may take up the active principles present in the blood circulation so as to decrease the free concentration thereof and, consequently, the toxicity thereof. Subsequently, these active principles would be gradually released with the degradation of the carrier, at concentrations below the toxic free concentrations.

Therefore, a further object of the present invention is the use of the nanocapsules of the present invention for the preparation of a medicament for taking up hydrophobic molecules present in the blood circulation subsequent to an instance of poisoning.

### 4) Sterilizing filtration

Finally, the nanocapsules according to the present invention have the advantage that they can be sterilized by sterilizing filtration through a filter with a diameter of 0.45 µm and even of 0.22 µm, this step possibly taking place directly after the final quenching step of the method described below. In fact, the applicant has noted that the nanocapsules according to the invention are sufficiently solid in suspension to be passed through this type of filter without undergoing any major modification and especially without losing their stealth properties and biodegradability. This characteristic allows said nanocapsules to be readily and safely employed for parenteral use, which makes them a carrier of choice for hospital-based or industrial mass use for example.

### 5) Use in dry/filtration form

The nanocapsules in accordance with the invention may advantageously be lyophilized and then reconstituted in the form of a colloidal suspension. They may thus be easily conserved for sustained periods of time. A suspension of nanocapsules according to the invention may in fact be reconstituted from a dry form extemporaneously, just before use. Such a lyophilization may take place directly after a sterilizing filtration step as described above.

The lyophilization of said nanocapsules consists in removing the water from the preparations by a phenomenon of sublimation. A suitable amount (approximately 5% m/m) of a cryoprotective or lyoprotective agent such as mannitol or trehalose, for example, may then be added to the suspension of nanocapsules to be lyophilized. After these compounds have completely dissolved, the suspensions chosen undergo a first step of rapid freezing at around -50°C. These suspensions are then lyophilized by direct passage of the water in the form of steam at low temperature under reduced pressure. The nanocapsules in dry form may then be stored in sterile form for long periods of time before use.

### 6) Other administrations

It is possible to envisage using the nanocapsules in accordance with the invention, as a carrier for active principles, for treating disorders of types other than tumors or inflammations. Specifically, one of the qualities of this colloidal carrier is its ability to evade the host's immune system and in particular the cells of the MPS, such as macrophages. It is therefore possible to envisage using the stealth nanocapsules of the invention in areas of the organism where the use of conventional carriers is particularly unsuitable. Such macrophage-rich areas exist in the pulmonary alveoli and the lymph nodes for example. It is thus possible to envisage administering said carrier via the routes for which phagocytosis and/or opsonization constitute a limit to the effectiveness of the pharmaceutical form.

### METHODS FOR PREPARING THE NANOCAPSULES

The nanocapsules constituting the stealth colloidal carrier in accordance with the invention may advantageously be synthesized using two methods. The first method is "conventional" since it is commonly used for synthesizing lipid nanocapsules. The second method has been developed by the applicant and is suitable for the nanocapsules according to the invention. It in particular makes it possible to obtain levels of incorporation of "pegylated" phospholipids which are greater than those obtained using the conventional method and also offers the possibility of incorporating phospholipids coupled to ligands for providing active targeting. These two methods are detailed successively below.

### 1) CONVENTIONAL METHOD FOR SYNTHESIZING THE NANOCAPSULES

The method for synthesizing the stealth colloidal carrier in accordance with the invention is advantageously free of any organic solvent. Moreover, it is a simple and relatively rapid method which does not require any particular material. In addition, this method only uses biodegradable compounds approved for parenteral use and able to be naturally assimilated by the organism, via a phenomenon of "bioresorption" in particular. Finally, the method in accordance with the invention makes it possible to synthesize nanocapsules of defined diameter chosen by the user within a diameter range varying from 40 to 200 nm. Adjusting the size of the nanocapsules synthesized will make it possible to adapt the carrier to the various types of tumor to be treated.

First of all, an oil/water emulsion is constituted from water, salt, the hydrophilic surfactant, the lipophilic surfactant, the fatty substances required for constituting the core of said nanocapsules, the molecules of lipids coupled to the molecules of PEG and the active principle(s) intended to be transported by the nanocapsules according to the invention. The active principle will be dissolved beforehand in the oily fatty phase which must constitute the core of the nanocapsules in accordance with the invention.

The first step therefore consists in weighing all the constituents and in heating them to a temperature greater than the melting temperature of the lipophilic surfactant, with gentle stirring, for example magnetic stirring, until a homogeneous oil/water emulsion is obtained. The mixture is thus initially brought to 65°C when the lipophilic surfactant is represented by HSPC.

The phase inversion of this oil/water emulsion is then brought about so as to transform it into a water/oil emulsion. To do this, the temperature of the mixture is increased to a temperature T2 greater than the phase inversion temperature (TIP) of the mixture thus constituted. The temperature of the mixture is then decreased to a temperature T1.

The phase inversion may optionally be followed by the disappearance of the conductivity of the formulation when the water/oil emulsion forms, but may generally be visible to the naked eye as described below. T1 is a temperature at which the conductivity is at least equal to 90-95% of the conductivity measured at 20°C. T2 is the temperature at which the conductivity disappears.

This operation is repeated by performing at least one cycle of temperature modification around the phase inversion zone between T1 and T2 until a translucent solution appears. Specifically, the organization of the system in the form of nanocapsules is reflected visually by a change in appearance of the initial system, which changes from opaque-white to translucent-white. This change occurs when the temperature falls below the phase inversion temperature (TIP). This temperature is generally between 6 and 15°C below the TIP.

The number of cycles applied to the emulsion depends on the amount of energy required to form the nanocapsules. The phase inversion between the oil/water emulsion and the water/oil emulsion results in a decrease in the conductivity when the temperature increases, until it disappears. The mean temperature of the phase inversion zone corresponds to the phase inversion temperature (TIP).

The oil/water emulsion may then be quenched, i.e. abruptly cooled, so as to obtain stable nanocapsules. This operation takes place with magnetic stirring, by diluting the emulsion between 3- and 10-fold using deionized water at 2°C +/- 1°C cast rapidly into the fine emulsion. This quenching step makes it possible to take the nanocapsules from the emulsion form, for which the lipids which make them up are fluid, to the suspension form, for which they are in a more set and therefore structurally very stable state. The particles thus obtained are stirred for 5 minutes. In the case of an industrial application, it is possible to envisage performing abrupt cooling of the preparation by circulating it in a system of thermal exchangers for example. In this particular case, dilution of the preparation is avoided.

Advantageously, the fatty phase is Labrafac^{®} cc, the lipophilic surfactant is HSPC (hydrogenated soy phosphatidylcholine) and the hydrophilic surfactant is Solutol^{®} HS15. The amphiphilic derivative of PEG is the pegylated phospholipid DSPE-PEG₂₀₀₀ or -PEG₅₀₀₀. Under these conditions, T1 = 60°C, T2 = 85°C and the number of cycles is equal to 3.

In terms of the constituents of the envelope of the nanocapsules, the molar percentage of the lipophilic surfactant which is lipid in nature is between 5 and 30 mol%. It is between 60 and 90 mol% for the surfactant which is hydrophilic in nature and between 0.5 and 5 mol% for the pegylated phospholipid.

An advantageous example of the molar percentage of the various constituents of the outer lipid envelope of said nanocapsules may be as follows:
- HSPC: 15.46 mol%
- Solutol^{®} HS15: 81.2 mol%
- DSPE-PEG₂₀₀₀: 3.34 mol%

In this example, the concentration of salt of the initial emulsion is 4.4% (m/m), and the mass of Labrafac^{®} cc constituting the core of the lipid nanocapsules represents 44% (m/m) of all the constituents which are lipid in nature or surfactants present in the preparation. This set of constituents will subsequently be referred to as total lipids (components other than water and sodium chloride); this term in particular includes the pegylated phospholipid. The ratio of the constituents of the outer lipid surface (mentioned above) to the total lipids is therefore 56%.

In general, the higher the mass ratio of the lipids making up the outer envelope of said nanocapsules to the total lipids constituting them, the smaller the nanocapsules. Thus, the size of the nanocapsules according to the invention can easily be modulated at the time they are synthesized, by choosing a suitable mass ratio of the lipids of the envelope to the total lipids.

Conversely, the size of the particles increases when the proportion of lipids constituting the core thereof increases.

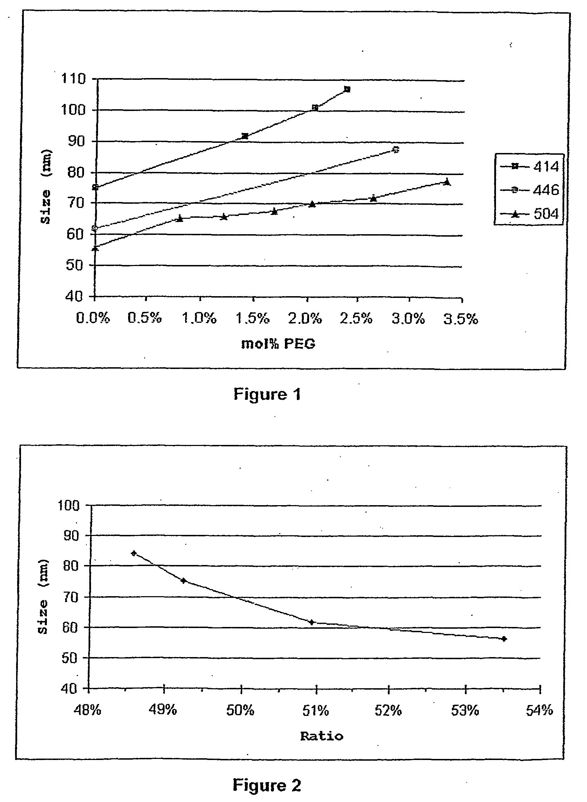

Moreover, the size of the particles decreases when the proportion of hydrophilic surfactant increases and when the proportion of (hydrophilic and lipophilic) surfactants increases. The surfactant causes a decrease in the interfacial tension and therefore stabilization of the system, which promotes the production of small particles. Specifically, the lower the interfacial tension, the more stable the suspension of lipid nanocapsules in an aqueous solution. For fixed amounts of hydrophilic and lipophilic surfactants and also of fatty acid esters, the addition of increasing amounts of pegylated lipids results in an increase in the size of the particles. It is possible to obtain various size ranges and various pegylated phospholipid contents by choosing given amounts of Solutol^{®} HS15 and DSPE-PEG (reference should be made to Figure 1 of Example 4).

According to a preferred embodiment, the fatty phase is Labrafac^{®} cc, the lipophilic surfactant which is lipid in nature is HSPC (Northern Lipids), the nonionic hydrophilic surfactant is Solutol^{®} HS15 and the lipid coupled to the molecules of PEG is DSPE-PEG₂₀₀₀. These compounds have the following characteristics:
- Labrafac^{®} cc (Gattefossé, Saint-Priest, France). It is an oil composed of caprylic/capric medium chain triglycerides (C₈ and C₁₀). The density thereof is 0.930 to 0.960 at 20°C. The HLB index thereof is about 1. The composition thereof is identical to Miglyol^{®} 812N (Sasol, Germany). Labrafac^{®} cc is included in the composition of preparations for parenteral nutrition.
- Solutol^{®} HS15 (BASF, Ludwigshafen, Germany). It is a poly(ethylene glycol)-660 12-hydroxystearate. It therefore plays the role of hydrophilic surfactant in the formulation. It can be used by injection (in mice by IV LD 50 > 3.16 g/kg, in rats 1.0 < LD 50 < 1.47 g/kg).
- DSPE-PEG₂₀₀₀ (Northern Lipids Inc., Vancouver, Canada). It is N-(carbamoylmethoxy poly(ethylene glycol) 2000)-1,2-distearoyl-sn-3-phosphoethanolamine in the form of sodium salt. It confers on the lipid nanocapsules their stealth properties. The long chains of poly(ethylene glycol) make it possible to protect the surface of the nanocapsules, via their steric effect, by preventing the proteins of the blood circulation from being deposited thereon (opsonization). It can be used by injection and is included in the liposomal preparation Doxil^{®} (Alza, Mountain View, USA).

The aqueous phase of the initial oil/water emulsion may also contain 4 to 8% of a salt such as sodium chloride. Modification of the salt concentration leads to a shift of the phase inversion zone. The higher the salt concentration, the lower the phase inversion temperature. This phenomenon is advantageous for the encapsulation of hydrophobic thermosensitive active principles. The incorporation thereof may then take place at a lower temperature.

The diameter of the nanocapsules is advantageously adjusted by adjusting the proportions of salt and of hydrophilic surfactant, and the purity of the lipophilic surfactant, in the starting mixture of the method of synthesis.

### 2) METHOD FOR SYNTHESIZING THE STEALTH NANOCAPSULES BY POST-INSERTION

The stealth lipid nanocapsules which are subjects of the present invention are advantageously prepared according to a method by post-insertion.

This method comprises a step of preformation of nanocapsules lacking amphiphilic derivative of poly(ethylene glycol), and then a step of post-insertion of said amphiphilic derivatives of poly(ethylene glycol) into the surface of these nanocapsules.

According to this method, said preformation step advantageously comprises the synthesis of nanocapsules lacking the amphiphilic derivative of PEG, according to the phase inversion of an oil/water emulsion brought about by several cycles of increase and decrease in temperature, as described above in the conventional method.

The method developed by the applicant is also free of any organic solvent and makes it possible to obtain a better yield of incorporation of "pegylated" phospholipids at the surface of the nanocapsules in accordance with the invention.

For example, it is possible to obtain nanocapsules in which the molar proportion of DSPE-PEG relative to the lipids of the envelope will be greater than 5 mol%, and preferentially of the order of 10 mol%. Such a proportion confers on the nanocapsules according to the invention good stealth properties. Now, it is difficult to obtain such proportions according to the conventional method of synthesis described above. Moreover, the method of synthesis by preformation of nanocapsules without PEG followed by the subsequent incorporation of DSPE-PEG by insertion makes it possible to precisely adjust the size of the nanocapsules, and thus to adapt them to the type of tumors to be treated by adjusting the proportion and the length of the hydrophilic chains of the amphiphilic derivative. Besides the insertion of a greater proportion of molecules of DSPE-PEG, it is possible to envisage, using this post-insertion technique, to incorporate derivatives of phospholipids comprising ligands at the end of a pegylated chain. These ligands may confer on the nanocapsules in accordance with the invention properties of active and specific targeting of cells of interest (solid and circulating tumors). This post-insertion method is particularly suitable for thermosensitive molecules since it is carried out at a temperature which does not exceed 60°C.

For example, nanocapsules based on HSPC, but lacking DSPE-PEG, are prepared beforehand in accordance with the method described above.

Said post-insertion step advantageously comprises a first step of coincubation of the preformed nanocapsules in the presence of the amphiphilic derivative of poly(ethylene glycol), and then a second "quenching" step during which the amphiphilic derivative of poly(ethylene glycol)/preformed nanocapsules mixture thus obtained is abruptly cooled so as to reach a temperature of between 0 and 5°C.

For example, water is added to the preparation containing the nanocapsules lacking DSPE-PEG described above so as to bring it to a precise volume in a volumetric flask. Thus, the precise concentration of the lipids in mg/ml is known and may be used as a basis for calculating the amounts of DSPE-PEG₂₀₀₀ or -PEG₅₀₀₀, for example, to be added in order to attain, for example, 6 mol% relative to all the lipids (including DSPE-PEG) making up the composition of the outer envelope of the lipid nanocapsules of the invention. Advantageously, the preparation of nanocapsules and the micellar solutions of DSPE-PEG are, subsequently, brought to and maintained at, separately, 60°C for a period of 15 minutes.

The step of coincubation of the amphiphilic derivative of poly(ethylene glycol)/preformed nanocapsules mixture is carried out at a temperature very slightly higher than the gel/liquid phase transition temperature of said lipophilic surfactant which is lipid in nature, but lower than the phase inversion temperature of the amphiphilic derivative of poly(ethylene glycol)/preformed nanocapsules mixture, in order to avoid any disorganization of the system due to the phase inversion phenomenon. Referring back to the example above, it is noted that, at 60°C, the surface of the lipid nanocapsules is much more fluid and allows the insertion of additional phospholipids. The required volume of micellar solution of DSPE-PEG is then introduced into the preparation of nanocapsules. This is allowed to incubate for 1 h 30 at 60°C, stirring vigorously every quarter of an hour. At the end of this incubation period, the preparations are immersed in an ice bath for one minute so as to abruptly set the envelope of the nanocapsules and to trap therein the molecules of DSPE-PEG inserted.

This post-insertion method makes it possible to obtain nanocapsules in accordance with the invention, the DSPE-PEG content of which is higher than that which it is possible to attain using the conventional method. In fact, the nanocapsules thus prepared exhibit, *in vivo,* a much greater furtive nature compared with that which it is possible to obtain with the nanocapsules prepared according to the conventional method.

It is also possible to carry out the post-insertion of DSPE-PEG at lower temperatures (37-50°C) but for longer incubation times. Thus, this method of insertion at low temperature may be suitable for the incorporation of derivatives of pegylated phospholipids which have, at the end of the PEG chain, thermosensitive groups such as certain oligopeptides and proteins for example. The latter compounds may be used as direction groups for the preparation of nanocapsules in accordance with the invention but allowing active targeting of tumor cells.

In addition, said post-insertion method makes it possible to introduce, in addition to the molecules of DSPE-PEG, activated phospholipids of the PDP-PEG₂₀₀₀-DSPE (pyridyldithiopropionylamino-PEG₂₀₀₀-DSPE) or MPB-PEG₂₀₀₀-DSPE (p-(maleimidophenyl)butaroylamino-PEG₂₀₀₀-DSPE) type (Northern Lipids Inc., Vancouver, Canada) which comprise reactive functions at the end of the pegylated chains. These reactive functions allow the coupling, once the nanocapsules according to the invention have been obtained, of compounds which are peptide in nature and which comprise thiol groups, such as oligopeptides or proteins of the antibody type, in order to confer on them active targeting properties.

### FIGURES

In the examples described below, reference will be made to the following figures:
- **Figure 1****:** Evolution of the size of nanocapsules prepared using HSPC, as a function of the molar percentage of DSPE-PEG₂₀₀₀
- **Figure 2****:** Evolution of the size of nanocapsules prepared using HSPC, lacking DSPE-PEG, as a function of the surface lipids/total lipids ratio
- **Figure 3****:** Evolution of the size of nanocapsules for various formulations obtained using the post-insertion method
- **Figure 4****:** Comparison of the stealth properties (kinetics of disappearance in the blood) for formulations of nanocapsules prepared using DSPE-PEG₂₀₀₀ or DSPE-PEG₅₀₀₀
- **Figure 5****:** Comparison of the stealth properties (kinetics of disappearance in the blood) of various formulations of nanocapsules prepared using DSPE-PEG₂₀₀₀ or DSPE-PEG₅₀₀₀, or of nanocapsules prepared according to the prior art
- **Figure 6****:** Comparison of the stealth properties (kinetics of disappearance in the blood) of various formulations of nanocapsules prepared using the conventional method or using the post-insertion method
- **Figure 7** **:** Evolution of the relative average body weight of mice after three intravenous injections of docetaxel (arrows). Values represent mean relative body weight, seven mice per group.
- **Figure 8** **:** Average number of days to reach 15 % body weight loss for different formulations of docetaxel administrered to mice (n=7).
- **Figure 9** **:** Accumulation of docetaxel loaded lipid nanocapsules (expressed as % injected [³H] *i.e.* the carrier) in C26 tumors at different time after intravenous administration to mice (n=5).
- **Figure 10** **:** Accumulation of docetaxel (ng) in C26 tumors at different time after intravenous administration to mice (n=5).

### A) EXAMPLES OF PREPARATION OF STEALTH NANOCAPSULES ACCORDING TO THE CONVENTIONAL METHOD

In the following examples:
Mass % = mass percentage relative to the total weight of the preparation
% w/w = mass percentage relative to the total weight of the nanocapsule
Mol% = molar percentage relative to the outer envelope of the nanocapsule
m%/env. = mass percentage relative to the weight of the outer envelope of the nanocapsule

### Example 1: Preparation comprising DSPE-PEG₂₀₀₀

Production of a preparation comprising DSPE-PEG₂₀₀₀ at 2.86 mol% for a total of approximately 1 gram of lipids, according to the conventional method.

The constituents required for synthesizing the nanocapsules in accordance with the invention are introduced into a 20 ml scintillation vial, in the amounts given in Table 1.

**Table 1**

| | **Mass (mg)** | **Mass %** | **Number of moles** | **Mol%** | **m%/env.** |
|---|---|---|---|---|---|
| HSPC | 75 | 1.50% | 9.843E-05 | 16.98% | 13.25% |
| DSPE-PEG | 46 | **0.92%** | 1.658E-05 | **2.86%** | **8.13%** |
| Solutol HS15 | 445 | 8.90% | 4.645E-04 | 80.15% | 78.62% |
| Labrafac | 504 | 10.08% | | | |
| NaCl | 220 | 4.40% | | Total lipids | 1070 |
| Water | 3710 | 74.20% | | Env./Total | 52.90% |
| Total | 5000 | 100.00% | | | |

In general, during weighing, maximum variations (relative to target values) of ± 1.5% for the lecithin and the DSPE-PEG, of ± 0.2 to 0.5% for the hydrophilic surfactant (Solutol^{®} HS15) and the triglycerides, and of ± 3% for the salt, are permitted. The amount of water introduced is at least equal to the value given and at most greater than the value given by 2%. In fact, as was explained above, the sizes of the nanocapsules obtained depend mainly on the ratio of the sum of the lipids constituting the envelope of the particles to the total mass of lipids *(EnvelopelTotal).*

After the weighing, the temperature is gradually increased to 60°C and the stirring is maintained for a few minutes, during which it is ensured that the aggregates of lecithin or of DSPE-PEG have disintegrated. The temperature is then increased to 80-85°C. Subsequently, the vial is transferred onto a non-heated plate and the temperature is allowed to drop, still with stirring, to 60°C. Three cycles of increase in temperature to 80°C and decrease to 60°C can then be undertaken. During the second increase in temperature, it is possible to observe the phase inversion phenomenon (below the phase inversion temperature (PIT), the solution is translucent, and it then becomes opaque and has a creamy appearance above the PIT).

At the end of the third cycle, quenching is carried out by adding a volume of cold water at 1 °C (12.5 ml) when the temperature falls by 10°C below the temperature at which the change in appearance occurred, as observed during the second and during the third increase in temperature. Following the quenching, the stirring is maintained for a period of 5 minutes. In the example cited, the temperature at which the change in appearance occurred is 75°C, the maximum temperature to which the preparation was taken is 82°C and the quenching was carried out at 62°C.

Subsequently, analysis of the size distribution profile by dynamic light scattering may be carried out after filtration of the preparation through a filter with a porosity of 0.2 µm. The preparation cited as an example makes it possible to obtain nanocapsules in accordance with the invention which are 87.5 ± 14 nm in size. The DSPE-PEG₂₀₀₀ content is 2.86 mol% relative to all the constituents of the surface. The other proportions were reported previously in Table 1.

The maximum proportion of DSPE-PEG₂₀₀₀ which it is possible to attain using this conventional preparation method is 3.34 mol%. The addition of a further amount of this pegylated phospholipid results in the instability of the preparations obtained following the final quenching.

### Example 2: Preparation comprising DSPE-PEG₅₀₀₀

Production of a preparation comprising DSPE-PEG₅₀₀₀ at 1.41 mol% for a total of approximately 0.6 gram of lipids, according to the conventional method.

The procedure is identical to that described in the previous example, with the exception of the amounts used, which are given in Table 2 below:

**Table 2**

| | **Mass (mg)** | **Mass %** | **Number of moles** | **Mol%** | **m%/env.** |
|---|---|---|---|---|---|
| HSPC | 37.5 | 1.50% | 4.921 E-05 | 15.54% | 11.89% |
| DSPE-PEG | 25.8 | **1.03%** | 4.461 E-06 | **1.41%** | **8.18%** |
| Solutol HS15 | 252 | 10.08% | 2.630E-04 | 83.05% | 79.92% |
| Labrafac | 252 | 10.08% | | | |
| NaCl | 116 | 4.64% | | Total lipids | 567.3 |
| Water | 1816.7 | 72.67% | | Env./Total | 55.58% |
| Total | 2500 | 100.00% | | | |

For this preparation, the opaque/translucent change in appearance occurs around 72°C, the maximum temperature reached during the cycles is 81 °C and the quenching was carried out below 65°C with a volume of 6 ml of iced water.

The mean size of the nanocapsules obtained, by dynamic light scattering, is 63.2 ± 9.83 nm after filtration of the preparation through a filter with a porosity of 0.2 µm.

The maximum proportion of DSPE-PEG₅₀₀₀ which it is possible to attain using this conventional preparation method is 1.49 mol%.

Thus, the amount of phospholipids incorporated using the conventional method is all the more limited since these molecules have longer PEG chains.

### B) EXAMPLES OF PRODUCTION OF NANOCAPSULES ACCORDING TO THE INVENTION BY POST-INSERTION

### Example 3: Preparation of nanocapsules according to the method of insertion of DSPE-PEG into preformed nanocapsules

Table 3 gives an example of amounts of the various constituents, making it possible to obtain formulations of lipid nanocapsules having diameters of around 80 nm, using the technique of insertion of DSPE-PEG into preformed nanocapsules.

The parent preparation of preformed nanocapsules without DSPE-PEG is prepared according to the conventional method. The amounts introduced are given in Table 3. After the quenching, the preparation is transferred into a volumetric flask and the volume is made up to 25 ml with distilled water.

**Table 3**

| | **Mass (mg)** | **Mass %** | **Number of moles** | **Mol%** | **m%/env.** |
|---|---|---|---|---|---|
| HSPC | 75 | 1.50% | 9.843E-05 | 17.48% | 14.42% |
| DSPE-PEG | 0 | **0.00%** | 0.00 | **0.00%** | **0.00%** |
| Solutol HS15 | 445 | 8.90% | 4.645E-04 | 82.52% | 85.58% |
| Labrafac | 504 | 10.08% | | | |
| NaCl | 220 | 4.40% | | Total lipids | 1024 |
| Water | 3756 | 75.12% | | Env./Total | 50.78% |
| Total | 5000 | 100.00% | | | |

Micellar solutions of DSPE-PEG₂₀₀₀ and DSPE-PEG₅₀₀₀ were prepared beforehand by weighing 75 mg of "pegylated" phospholipids, adding a sufficient amount of distilled water to produce dissolution and making the volume up, after total dissolution, to a precise total volume of 5 ml.

2-ml volumes of parent preparation are then distributed into stoppered haemolysis tubes which are placed in a water bath at 60°C. The micellar solutions are also placed in the water bath. The preparations of lipid nanocapsules and the micellar solutions of DSPE-PEG are preincubated separately at 60°C for 15 minutes. After this period of time, the required volumes are added so as to obtain a theoretical proportion of 6 mol% of phospholipids associated with molecules of PEG in the envelope of the nanocapsules. Thus, with the volumes and the concentrations of the micellar solutions given above, 532 µl of DSPE-PEG₂₀₀₀ or 1108 µl of DSPE-PEG₅₀₀₀ are added. A control is also prepared by adding 800 µl of hot water.

After these amounts have been added, the solutions are left for 1 h 30 at 60°C and vortexed every quarter of an hour. After this incubation period, the preparations are plunged into an ice bath.

The sizes of the lipid nanocapsules obtained are given in Table 4.

**Table 4**

| **Preparation** | **Nanocapsule size (nm)** |
|---|---|
| Non-incubated parent preparation | 69 ± 12.9 |
| Heated control | 69.9 ± 14.2 |
| DSPE-PEG₂₀₀₀ | 77.9 ± 14 |
| DSPE-PEG₅₀₀₀ | 78.4 ± 13 |

The control preparation, incubated under the same conditions, has particles with a size close to those of the parent preparation not incubated at 60°C. The preparations having been incubated in the presence of DSPE-PEG exhibit a substantial increase in the mean diameter of the particles, suggesting the insertion of the "pegylated" phospholipids at the surface of the nanocapsules.

### C) INFLUENCE OF VARIOUS PARAMETERS ON THE SIZE OF THE NANOCAPSULES IN ACCORDANCE WITH THE INVENTION

### Example 4: Control of the size of nanocapsules prepared according to the conventional method

The preparations are produced in accordance with the conventional method as described in Example 1. The preparations are produced with an amount of lipids of around 1 g. Figure 1 represents the evolution of the size of the nanocapsules, as a function of the proportion of DSPE-PEG₂₀₀₀ added, for amounts of hydrophilic surfactant, Solutol^{®} HS15, fixed, respectively, at 414 mg (413.5 ± 0.50), 446 mg (445.85 ± 0.01) or 504 mg (504.4 ± 0.27). The amounts of HSPC, Labrafac^{®} cc and NaCl are maintained constant for all the preparations, respectively at 76 mg, 504.5 mg and 220 mg.

In general, the size increases for decreasing amounts of Solutol^{®} HS15. In the same series, for a given amount of Solutol^{®} HS15, the size increases with the increasing proportions of DSPE-PEG added.

### Example 5: Control of the size for nanocapsules prepared according to the post-insertion method

When the intention is to obtain particularly long circulation times or to introduce ligands, it is necessary to make use of the post-insertion method as described in Example 3. Thus, nanocapsules are generated beforehand, which are free of DSPE-PEG and for which it is possible to adjust the size by varying the mass ratio of the total of the lipids and surfactants constituting the surface of the particles to the total of all the lipids and surfactants making up the composition of the preparation (surface + core). The relationship between this surface lipid/total lipid ratio and the size of the nanocapsules obtained, suitable for the post-insertion method, is shown in Figure 2.

The formulations shown in Figure 2 were prepared for an amount of approximately 1 g of lipids. The amounts of HSPC, Labrafac^{®} cc and NaCl are constant and correspond to those used in Example 4. The amounts of Solutol^{®} HS15 used and the corresponding surface lipids/total lipid ratios (S/T ratio) are given in Table 5 below.

**Table 5**

| **Solutol^{®} HS15 (mg)** | **S/T ratio** |
|---|---|
| 400.6 | 48.6% |
| 413.2 | 49.2% |
| 445.9 | 50.9% |
| 504.2 | 53.5% |

Subsequently, it is possible to introduce the pegylated phospholipid DSPE-PEG using the post-insertion method, in order to obtain the stealth nanocapsules in accordance with the invention. The post-insertion method is carried out according to Example 3 for a final total proportion of 6 mol% of DSPE-PEG 2000 or 5000.

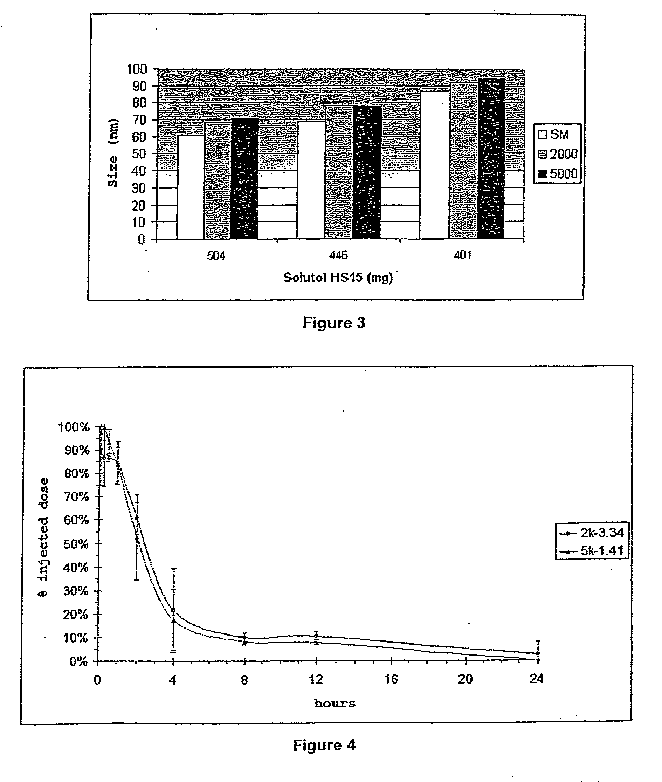

The sizes obtained for the parent formulations (series SM), free of DSPE-PEG, having respectively 504, 446 and 401 mg of Solutol^{®} HS15, and the daughter formulations in accordance with the invention, having been the subject of incorporation of DSPE-PEG 2000 and 5000 using said post-insertion method, are represented in Figure 3.

Thus, the insertion of DSPE-PEG₂₀₀₀ results in an increase in the diameter of 7.2 nm on average. The increase in size is more marked for the DSPE-PEG₅₀₀₀, which exhibits a mean increase of 9.1 nm in the diameter of the particles.

On the basis of these increases in diameter, which reflect the effective insertion of pegylated phospholipids at the surface of the lipid nanocapsules, and in the knowledge of the evolution of the size of the nanocapsules free of PEG as a function of the surface/total lipids ratio (Figure 2), it is possible to adjust the size of the nanocapsules in accordance with the invention so as to facilitate the extravasation through the fenestrations present in the endothelium of particular tumor capillaries.

### D) RESULTS OF CLEARANCE EXPERIMENTS CARRIED OUT IN VIVO WITH THE STEALTH NANOCAPSULES IN ACCORDANCE WITH THE INVENTION

### Example 6: Kinetics of disappearance of the carrier (PEG₂₀₀₀/PEG₅₀₀₀ comparative data)

The blood clearance of the nanocapsules in accordance with the invention was evaluated in a comparative study between a formulation of nanocapsules based on 3.34 mol% of DSPE-PEG₂₀₀₀ ("2k-3.34" curve) and a formulation of nanocapsules based on DSPE-PEG₅₀₀₀, the molar proportion of DSPE-PEG₅₀₀₀ of which is 1.41 mol% ("5k-1.41"). These formulations were prepared according to said conventional method as described in Examples 1 and 2.

To do this, solutions of nanocapsules in accordance with the invention were injected intravenously into male rats (Sprague-Dawley, 325 g) in a proportion of 4 rats per formulation. The injected doses of lipids were chosen so as to be approximately 10 times less than the doses starting from which phenomena of "saturation of the MPS" may take place (which would result in sustained circulation times due to the capacity for purging being overrun). Thus, a solution of nanocapsules comprising a total amount of lipids of 2 mg in a volume of 400 µl was injected into each rat.

The nanocapsules in accordance with the invention were labelled beforehand with a tritiated radioactive tracer: [³H]-cholesterol hexadecyl ether which is a nonexchangeable label. The dose of radioactive label represents 3 microCurie (µCi) per rat. Nine successive blood samples were taken after injection of the nanocapsules, at 5, 15 and 30 minutes and then at 1, 2, 4, 8, 12 and 24 hours.

The blood samples are digested in the presence of Soluene 350 (Canberra Packard, Mississauga, Canada) for one hour at 50°C. After digestion, the blood samples are decolored using 30% aqueous hydrogen peroxide. The scintillation fluid Hionic Fluor (Canberra) is then added and the samples are left to stand overnight. The amount of labelled nanocapsules present in the blood at the various times is then assayed by counting the radioactivity using a scintillation counter. Assaying controls makes it possible to determine the number of dpm (disintegrations per minute) corresponding to 100% of the dose. The total blood volume in rats is 75 ml/kg. Thus, it is possible to express the results as percentage of the total injected dose. The blood profiles obtained of this experiment are recapitulated in Figure 4.

Table 6 gives the name, the mean size and the molar proportion of DSPE-PEG incorporated, according to the conventional method, into the outer envelope of said lipid nanocapsules.

The nomenclature used reports the molar mass of the PEG component (2k, 5k for ₂₀₀₀ or ₅₀₀₀) and the molar percentage of DSPE-PEG relative to all the surface lipids.

**Table 6: Description of the formulations used**

| **Name** | **Size (nm)** | **DSPE-PEG** |
|---|---|---|
| 2k-3.34 | 77 ± 15 | 3.34 mol% |
| 5k-1.41 | 61 ± 15 | 1.41 mol% |

These results show that the plasmatic half-life of nanocapsules in accordance with the invention is between 2 and 3 hours in rats both for the nanocapsules based on DSPE-PEG₂₀₀₀ at 3.34 mol% and for the nanocapsules based on DSPE-PEG₅₀₀₀ at 1.41 mol%. However, it is important to note the greater stealth effect provided by the chains of DSPE-PEG₅₀₀₀. Specifically, for a number of moles which is more than two times less than the number of moles of DSPE-PEG₂₀₀₀ at the surface of the nanocapsules, the longer PEG chains in fact provide a protective effect comparable to that conferred by the chains having a mass of 2 000 g/mol.

### Example 7: Comparative study of the nanocapsules of the invention compared with the nanocapsules of the prior art

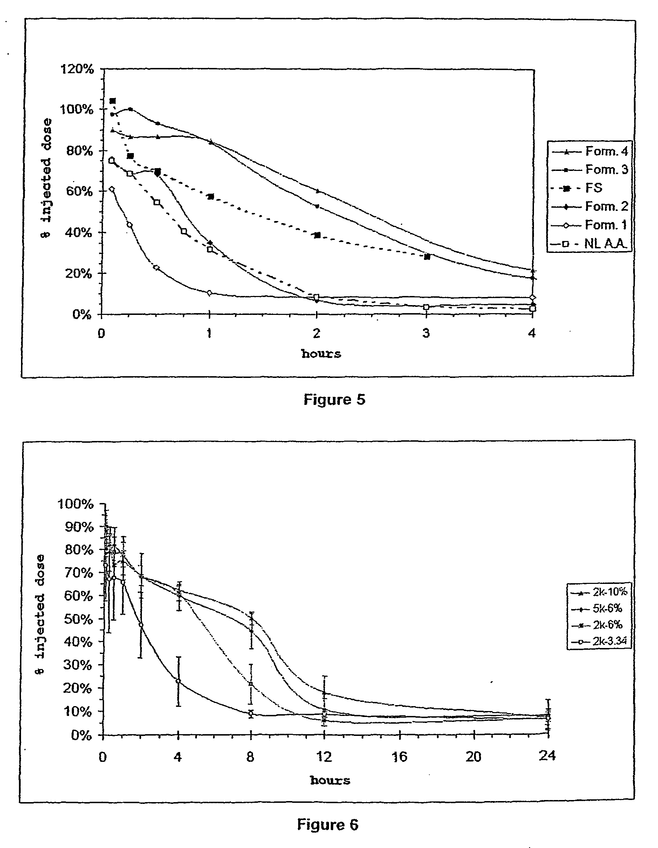

A study, similar to that shown in Example 6, aimed at comparing the rates of disappearance in the blood of the lipid nanocapsules in accordance with the invention and of the lipid nanocapsules as described in the prior art in patent No. WO 01/64328, was carried out. The results obtained for formulations 1 to 4 were obtained in rats under conditions identical to the results given in Figure 4. The formulations represented are briefly described below and the data obtained are reported in Figure 5. This figure also comprises, by way of comparison, the data obtained by Stolnik *et al*. (available for 3 hours of blood kinetics only) and also by Cahouet *et al*.
***Formulation 1*:** Conventional nanocapsules as described in patent No. WO 01/64328: the lipophilic surfactant is Lipoïd^{®} S75-3, which is not a pure lecithin (70% of phosphatidylcholine) and the particles lack PEG;
***Formulation 2*:** Nanocapsules based on Lipoïd^{®} S75-3, but comprising DSPE-PEG₂₀₀₀ (1.65 mol%) introduced according to the conventional method;
***Formulation 3*:** Nanocapsules based on HSPC (>99% of phosphatidylcholine) and on DSPE-PEG₅₀₀₀ (1.41 mol%), nanocapsules in accordance with the invention based on PEG₅₀₀₀ introduced according to the conventional method;
***Formulation 4*:** Nanocapsules-DSPE-PEG₂₀₀₀ (3.34 mol%), nanocapsules in accordance with the invention based on PEG₂₀₀₀, maximum amount which can be incorporated according to the conventional method for HSPC;
***FS*:** Stolnik formulation: micelles of PLA-PEG in the proportion 13:5 (the PEG component having a molar mass of 2 000 g/mol), the diameter of which is of the order of 75 nm. The data of the best formulation are taken from the graph contained in the published article (Stolnik *et al.,* J. Drug Target., **9**: 361, 2001). These data are also obtained following intravenous injection into rats of an amount of nanocapsules which does not saturate the MPS;
***NL A.A.:*** Conventional nanocapsules formulation of the prior art. These data were taken from the study by Cahouet et al. (Int. J. Pharm. 242:367 2002). These results were also obtained following intravenous injection (unknown dose) into rats of lipid nanocapsules prepared by the abovementioned authors according to a formulation based on Lipoïd^{®} S75-3 described in patent No. WO 01/64328.

Formulations 1 and 2 represent, respectively, formulations of conventional nanocapsules, the surface of which comprises Lipoid^{®} S 75-3 (lecithin comprising impurities), and these same nanocapsules, the surface of which also comprises 1.65 mol% of DSPE-PEG₂₀₀₀. This level is close to the maximum content which can be incorporated for nanocapsules comprising Lipoïd^{®} S 75-3, prepared according to the conventional method. The DSPE-PEG₂₀₀₀ content of formulation 4 is also at its maximum by virtue of the conventional method. The lack of impurities and the homogeneity of the phosphatidylcholine HSPC make it possible to incorporate such DSPE-PEG contents into the nanocapsules in accordance with the invention (practically twice as much as with Lipoïd^{®} S 75-3).

These results show that the nanocapsules in accordance with the invention (formulations 3 and 4) exhibit a significantly lower clearance than the lipid nanocapsules of the prior art (formulation 1, as described in patent No. WO 01/64328) which are very rapidly eliminated from the blood compartment. Thus, for formulation 1, only 10% of the injected dose is still present in the blood after one hour. The nanocapsules of the prior art therefore exhibit no stealth properties. Moreover, the incorporation of DSPE-PEG₂₀₀₀ into the nanocapsules of the prior art (formulation 2) only very slightly improved their persistence in the blood circulation: their half-life time is also less than one hour. In fact, the respective increases in the AUCs (Area Under the Curve between 5 minutes and 24 hours) relative to formulation 1 (AUC_{Form x}/AUC_{Form 1}) are:
- Formulation 2: 1.09
- Formulation 3: 2.31
- Formulation 4: 2.68.

Moreover, as mentioned in the description of the prior art, the nanocapsules in accordance with patent No. WO 01/64328 have also been the subject of an *in vivo* study in rats by Cahouet *et al.* The kinetics of elimination from the blood of these nanocapsules exhibits a profile (formulation NL A.A. in Figure 5) which is slightly superior to that obtained for formulation 1 and relatively similar to that of formulation 2, probably due to the difference in injected dose (NL A.A.: unknown dose). The half-life time of the nanocapsules of Cahouet *et al*. (NL A.A.) is less than one hour (precisely, 45 minutes). The profile obtained by the applicant for formulation 1 is thus relatively similar to that given in the study of the prior art. These two control formulations, which do not comprise any chain of PEG greater than 1 000 g/mol, show the ineffectiveness of the short PEG chains of Solutol^{®} HS15 (660 g/mol) in conferring protection against rapid elimination from the blood circulation. Thus, these two formulations do not exhibit any stealth properties nor a sustained circulation (10% of the injected dose at the 2nd hour) and may be considered to be non-stealth references for evaluating the effect of the modifications to the composition of the carriers (use of a lecithin with high phosphatidylcholine purity), in particular the addition of long PEG chains.

Figure 5 also represents the data of the prior art as given in the publication by Stolnik *et al.* (legend FS). Their formulation of PLA-PEG micelles exhibits a stealth nature with slightly less than 40% of the injected dose still present in the blood circulation after two hours. However, the stealth nature of this colloidal preparation is less than that of the nanocapsules in accordance with the invention, which exhibit more than 50% and more than 60% of the injected dose after the 2nd hour for formulations 3 (DSPE-PEG₅₀₀₀) and 4 (DSPE-PEG₂₀₀₀), respectively. Given that the proportion of nanocapsules in accordance with the invention (formulations 3 and 4) present in the blood circulation during the first 4 hours following the injection is clearly greater than that of the stealth nanoparticles of the prior art (formulation FS), the amount of carrier liable to reach solid and/or circulating tumor cells will advantageously be increased, thus increasing the chances of success of the treatment.

### Example 8: Potentiation of the stealth properties provided by the post-insertion method

An *in vivo* study, performed under the same conditions as the previous studies, was carried out with 4 different formulations. Thus, in order to evaluate the advantage of the post-insertion method, the applicant compared the stealth nature of three formulations prepared according to said post-insertion method, with the stealth nature of the best formulation prepared according to the conventional method. The four formulations which were the subject of this *in vivo* study are as follows:
**Formulation *2k-3.34:*** Nanocapsules-HSPC-PEG₂₀₀₀ (3.34 mol%), nanocapsules in accordance with the invention based on PEG₂₀₀₀, maximum amount which can be incorporated according to the conventional method for HSPC. This formulation corresponds to formulation 4 of Example 7, injected into a new group of 4 rats;
**Formulation *2k-6%:*** Nanocapsules based on HSPC having been the subject of incorporation of an amount of DSPE-PEG₂₀₀₀ corresponding to 6 mol% of the total lipids of the surface of the nanocapsules in accordance with the invention according to the post-insertion method, as detailed in Example 3;
**Formulation *5k-6%:*** Nanocapsules based on the same parent formulation comprising HSPC, prepared for the production of the nanocapsules of formulation 2k-6%, but having been the subject of incorporation of DSPE-PEG₅₀₀₀ by the post-insertion method, nanocapsules in accordance with the invention;
**Formulation** ***2k-10%:*** Nanocapsules based on the same parent formulation comprising HSPC, prepared for the production of the nanocapsules of formulation 2k-6%, but having been the subject of incorporation of 10 mol% of DSPE-PEG₂₀₀₀ by the post-insertion method, nanocapsules in accordance with the invention.

The results obtained for the elimination from the blood of the three formulations detailed above are given in Figure 6.

The data given in Figure 6 demonstrate the advantage of the post-insertion method. Specifically, said post-insertion method makes it possible to obtain nanocapsules in accordance with the invention, the stealth properties of which is clearly superior to that of nanocapsules in accordance with the invention but prepared according to the conventional method. Moreover, this study also demonstrates the stability and the conservation of the stealth properties, over time, of the nanocapsules in accordance with the invention. Formulation 2k-3.34 was prepared 3 months earlier and injected again for the present study. The profiles obtained are comparable (an amount slightly greater than 22% of the injected dose is found at the end of the 4th hour, as in Figure 4). The formulations shown in Figure 6 exhibit pronounced stealth properties. The formulations of control nanocapsules (without DSPE-PEG) or (formulation 1 of the preceding example and that of the study of the prior art) exhibit less than 10% of the residual dose after two hours.

The post-insertion method makes it possible to incorporate amounts of DSPE-PEG which are greater than those which can be incorporated according to the conventional method, as shown by the blood profiles of Figure 6, which makes it possible to improve the circulation time of the preparations and to adapt them to applications requiring greater proportions of nanocapsules in the blood during the first 12 hours following the injection thereof. Thus, the formulations prepared according to said post-insertion method all exhibit approximately 60% of the injected dose at the end of the 4th hour. At the 8th hour, the residual dose of nanocapsules of formulation 2k-6% is more than 18%. The same proportion of 6 mol% of DSPE-PEG, but having longer PEG chains (DSPE-PEG₅₀₀₀) makes it possible to obtain more than 40% of the injected dose at the 8th hour. When more DSPE-PEG₂₀₀₀ is introduced, up to 10 mol%, it is possible to obtain a profile slightly superior to that of the formulation 5k-6%, with 50% of the injected dose at the 8th hour. The stealth properties of the formulations of nanocapsules in accordance with the invention prepared according to the post-insertion method is thus clearly greater than that observed for "stealth" solid nanoparticles as described in the literature.

### E) EXAMPLES OF PREPARATION OF STEALTH NANOCAPSULES LOADED WITH ANTICANCER DRUGS

### Example 9: preparation of paclitaxel and docetaxel stealth lipid nanocapsules

Paclitaxel and docetaxel can be incorporated in lipid nanocapsules according to the conventional method and then the drug-loaded nanocapsules obtained can be pegylated via the post-insertion method to provide stealth properties.

A solution of docetaxel or paclitaxel in triglycerides, which will constitute the inner core of the lipid nanocapsules, is prepared by dissolving the drug into the triglyceride at 75°C. The amount of drug is adjusted in order to obtain solutions that will not recrystallize upon cooling to room temperature. In the case of paclitaxel and docetaxel, the triglycerides with short alkyl chains, demonstrate a better solubility than those with long alkyl chains (>C8). Solutions of paclitaxel and docetaxel in triglycerides, which vary in C8 ester composition, are presented in table 7.

**Table 7 : triglyceride solutions of paclitaxel and docetaxel**

| Triglyceride | | | Solubility (% w/w)* | |
|---|---|---|---|---|
| Name/supplier | Specifications | C8 content | Paclitaxel | Docetaxel |
| Labrafac^{®} CC | C8:50-80% | 56,7% | 1,6 | 2 |
| (Gattefossé) | C10:20-50% | | | |
| Miglyol^{®} 810 N | C8:65-80% | 70,4% | 2,7 | 3,9 |
| (Sasol) | C10:20-35% | | | |
| Tricaprylin | C8:>99% | >99% | 6 | 7 |
| (Sigma) | C10: NR | | | |

| | | | | |
|---|---|---|---|---|
| *percentage by weight of drug in the triglyceride solution obtained | | | | |

Table 7 shows that triglycerides with the higher C8 ester content (i.e glyceryl tricaprylate = tricaprylin) solubilized greater amounts of paclitaxel or docetaxel. Tricaprylin, the pure C8 compound, was the best solvent for both drugs. Solutions of 6% paclitaxel or less and 7% docetaxel or less in tricaprylin may be kept at room temperature for long period of time.

As an example, a 6% solution of docetaxel in tricaprylin has the following composition:
- Tricaprylin 552.0 mg
- Docetaxel 35.3 mg

The 6% solution of docetaxel in tricaprylin is stable for months at 25°C.

Drug loaded lipid nanocapsules, without DSPE-PEG, can be prepared from the solutions of paclitaxel or docetaxel in tricaprylin according to the conventional method. When tricaprylin solutions of paclitaxel 6% or docetaxel 7% are used to prepare lipid nanocapsules, drug crystals appear after 10 days or 118 days respectively. This destabilization phenomenon is due to the fact that these tricaprylin solutions are close to saturation. Stability of the drug loaded lipid nanocapsules preparations can be improved by decreasing the amount of drug in tricaprylin. Lipid nanocapsules prepared with paclitaxel 5% in tricaprylin are stable for 32 days and lipid nanocapsules prepared with docetaxel 6% in tricaprylin are stable for at least 7 months at 25°C or at 5°C.

Paclitaxel or docetaxel loaded lipid nanocapsules prepared with a 5% paclitaxel or a 6% docetaxel "solution of tricaprylin" may have the following composition:
- HSPC 75 mg
- Solutol^{®} HS 15400 mg
- "Solution of tricaprylin" 504 mg
- NaCl 220 mg
- Water qs ad 5000 mg

The drug loaded nanocapsules have the following characteristics (Table 8).

**Table 8: characteristics of drug loaded lipid nanocapsules**

| **Solution of Tricaprylin used** | **Drug loading (% w/w)** | **Size (nm)** |
|---|---|---|
| No drug | 0 | 72 |
| Paclitaxel 5% | 2.58% | 73 |
| Docetaxel 6% | 3.09% | 77 |

Docetaxel was preferred owing to better drug loading and improved stability than paclitaxel. However, 2% w/w paclitaxel lipid nanocapsules are stable for months. The stability of 3.09% w/w ("3%") docetaxel lipid nanocapsules is greater than 7 months at 25°C.

The parent drug loaded lipid nanocapsules preparation is then used to prepare drug loaded stealth lipid nanocapsules according to the post-insertion method as described in example 3. Briefly, the volume of the drug loaded lipid nanocapsules preparation obtained is made up to 25 mL. Aliquots of 2 mL are used for incubations (15 min pre-incubation followed by 90 min incubation at 60°C) with DSPE-PEG micellar solutions (see Table 9); the preparations are cooled in an ice bath at the end of the post-insertion time. The drug loaded lipid nanocapsules subject of the present invention obtained after DSPE-PEG post-insertion were stable for several months (no recrystallization of the incorporated drug nor significant variation in size were observed). Several DSPE-PEG post-insertions with lipid nanocapsules loaded with docetaxel 3.5% are reported in Table 9.

**Table 9: characteristics of docetaxel 3.5% (w/w) stealth lipid nanocapsules**

| **Preparation** | **Size (nm)** |
|---|---|
| Control LN* | 72 |
| LN-DSPE-PEG₂₀₀₀-6mol% | 78 |
| LN-DSPE-PEG₅₀₀₀-6mol% | 79 |
| LN-DSPE-PEG₂₀₀₀-10mol% | 82 |
| LN-DSPE-PEG₅₀₀₀-10mol% | 83 |

| | |
|---|---|
| *Lipid Nanocapsules (LN) containing 3.5% docetaxel, incubated in the same conditions without post-insertion | |

No recrystallization of docetaxel occurred in any of the preparations. The significant increase in diameter, compared to the control preparation, reflects the effective insertion of DSPE-PEG lipids at the surface of the docetaxel loaded lipid nanocapsules.

### F) RESULTS OF TOXICITY EXPERIMENTS CARRIED OUT IN VIVO WITH ANTICANCER DRUG LOADED STEALTH NANOCAPSULES IN ACCORDANCE WITH THE INVENTION

### Example 10: Toxicity assessment of docetaxel loaded nanocapsules versus Taxotere^{®} in healthy mice

The toxicity of docetaxel in mice was evaluated for various formulations at different dosages. Toxicity of anticancer drugs may be evaluated by measurement of the body weight loss of mice after intravenous injection of the formulations. Docetaxel was injected as the commercial injectable solution, Taxotere^{®} (Aventis)*, or formulated in non-pegylated Lipid Nanocapsules (LN) and Stealth Lipid Nanocapsules (S-LN).

*Taxotere^{®} is a solution of docetaxel in polysorbate 80, ethanol and water.

Lipid Nanocapsules and Stealth Lipid Nanocapsules were prepared in order to yield a 3% w/w docetaxel loading according to the method described in example 9. These preparations, which present more than 7 months of stability at 25°C, have the following characteristics (Table 10).

**Table 10 : characteristics of 3% w/w docetaxel loaded LN and S-LN**

| Name | Composition | Pegylation | Size |
|---|---|---|---|
| LN | HSPC 75 mg | - | 70 nm |
| S-LN | Solutol^{®} HS 15 400 mg | DSPE-PEG₂₀₀₀ 6 mol% | 80 nm |
| | "Solution of tricaprylin*" ..... 504 mg | | |

| | | | |
|---|---|---|---|
| *A docetaxel 6% w/w solution in tricaprylin was used | | | |

The docetaxel formulations (Taxotere^{®}, non-pegylated 3% w/w docetaxel-loaded nanocapsules and 3% w/w docetaxel-loaded DSPE-PEG₂₀₀₀-6mol% nanocapsules) were diluted with physiological saline and injected at 20, 40 and 60 mg/kg docetaxel. Due to the high toxicity of the injectable docetaxel solution, the 60 mg/kg dosage was not investigated for Taxotere^{®}. These formulations of docetaxel were administered to eight groups of mice, plus one other "control" group receiving injections of physiological saline (Table 11).

**Table 11: Formulations and dosages used for the toxicity study**

| **Formulation / dosage** | **20 mg/kg** | **40 mg/kg** | **60 mg/kg** |
|---|---|---|---|
| 3% w/w docetaxel loaded Lipid Nanocapsules (LN) | LN-20 | LN-40 | LN-60 |
| DSPE-PEG₂₀₀₀-6mol% 3% w/w docetaxel loaded Stealth Lipid Nanocapsules (S-LN) | S-LN-20 | S-LN-40 | S-LN-60 |
| Taxotere^{®} | TXT-20 | TXT-40 | - |

The toxicity study was undertaken in healthy female BALB/c mice weighing 17-20 g (7 mice per formulation, 8 formulations plus 1 control). Mice were administered 250 µL of a given formulation (or a solution of physiologic saline, for control group) as a single intravenous dose. The weight of the mice before the first injection (D0) was defined as reference weight. Mice were administered 3 doses on days 0 (D0), 5 (D5) and 10 (D10). Mice were weighed and checked daily for apparent adverse reactions up to D28 and then at D30, D33 and D40.

Figure 7 shows the evolution of the relative body weight (D/D0) for the nine groups of mice (error bars are not represented; all RSD were < 2.4%). All Docetaxel formulations induced body weight losses which increased with increasing dosage. The control group (Ctrl) showed an increase in weight. The study was interrupted for group TXT-40 on D10 before the third injection because 2 animals experienced atrophia and paralysis of the hind legs. The animals of group TXT-40 were sacrificed on day 10 because they lost more than 15% body weight and thus would not tolerate a third injection.

Docetaxel loaded into LN or S-LN at 20 mg/kg induced lower body weight losses (b.w.l.) at nadir (D23) than the corresponding dose of docetaxel in the Taxotere^{®} solution (P < 0.05, Student Test). Moreover, a 40 mg/kg dose of docetaxel in Taxotere^{®} (TXT-40) exhibited a high toxicity with 15% b.w.l. reached in 9.7 days; the b.w.l. were greater than the corresponding doses of LN-40 and S-LN-40 and even greater than the 60 mg/kg doses (LN-60 and S-LN-60). In order to compare the toxic effect of the formulations, the days, after which 10% and 15% b.w.l. were observed, are reported in Table 12 and figure 8 respectively.

**Table 12: Average day corresponding to 10% body weight loss in mice (n=7) and cumulated dose of Docetaxel received**

| **Group** | **Average Day ± SD** | **Cumulated dose (mg/kg)** |
|---|---|---|
| TXT-40 | 6.4 ± 1.13 | 80 |
| LN-60 | 8.3 ± 0.95 | 120 |
| S-LN-60 | 9.4 ± 0.79 | 120 |
| LN-40 | 10.7 ± 0.95 | 120 |
| S-LN-40 | 11.1 ± 2.41 | 120 |
| TXT-20 | 13.0 ± 1.53 | 60 |
| LN-20 | 16.1 ± 3.08 | 60 |
| S-LN-20 | 17.3 ± 3.33 | 60 |

Table 12 and figure 8 show that the Taxotere formulations induced body weight loss more rapidly than the nanocapsules formulations at equivalent dosages. The 15% b.w.l. occurred significantly sooner for TXT-40 than any of the formulations LN-40 and S-LN-40 or LN-60 and S-LN-60 (Student Test P < 0.05).

In conclusion, the encapsulation of Docetaxel in Lipid Nanocapsules or Stealth Lipid Nanocapsules was shown to be effective in reducing the toxicity of this anticancer agent compared to the commercial solution Taxotere^{®} administered intravenously at the same doses. Moreover, the administration of a cumulated dose of 80 mg/kg of Taxotere^{®} (2 x 40) was found to be highly toxic with severe side effects whereas it was possible to administer higher doses of docetaxel in LN or S-LN, i.e. 180 mg/kg (3 x 60) with less than 20% b.w.l. at nadir and a body weight increase a few weeks after the last dose. Thus, it is possible to administer higher doses of docetaxel, or to observe less toxic effects, when the drug is formulated in Lipid Nanocapsules or Stealth Lipid Nanocapsules *vs* a solution.

### G) RESULTS OF IN VIVO BIODISTRIBUTION STUDIES CARRIED OUT WITH ANTICANCER DRUG LOADED STEALTH NANOCAPSULES, IN ACCORDANCE WITH THE INVENTION, ADMINISTERED TO TUMOR BEARING MICE

### Example 11: Tumor accumulation of docetaxel loaded nanocapsules versus Taxotere^{®} in C26 tumor bearing mice

The tumor accumulation of anticancer drug loaded stealth lipid nanocapsules in accordance with the invention was evaluated in a comparative study between a formulation of 3% w/w docetaxel-loaded DSPE-PEG₂₀₀₀-6mol% Stealth Lipid Nanocapsules (S-LN), a formulation of non-pegylated 3% w/w Lipid Nanocapsules (LN) and the commercial solution of docetaxel, Taxotere^{®} (TXT).

### Preparation of the formulations

Docetaxel loaded LN and S-LN were prepared according to said conventional method and post-insertion method using the same ingredients and in the same relative proportions as described in example 10 (Table 10). Both nanocapsule formulations were radiolabeled with [³H]-cholesterol hexadecyl ether (to follow the nanocapsules) and [¹⁴C]-docetaxel (to follow the drug). [¹⁴C]-docetaxel was also used to label the commercial docetaxel solution Taxotere^{®}. The three formulations LN, S-LN and TXT were appropriately diluted with physiological saline and injected iv at a docetaxel dose of 1 mg/kg (150 µL) The nanocapsule groups received 0.7 µCi of [³H] and 0.2 µCi of [¹⁴C], and the Taxotere^{®} mice were injected with 0.35 µCi of [¹⁴C].

### C26 Tumor-bearing mice

Experiments were performed on female BALB/c mice (17-20 g) bearing Colon carcinoma 26 (C26) tumors. Before tumor implantation, hair on the back of the mice was removed by shaving and chemical depilation. Three C26 tumors were implanted on the back of each mouse by subcutaneous injection of 2 x 10⁶ cells suspended in 50 µL growth medium. Mice were tested 10 days after cell inoculation when tumor diameter reached 8 mm in average (5-10 mm). Tumor-bearing mice were then pooled in three groups (LN, S-LN and TXT) and injected intravenously with 150 µL of formulation corresponding to a dose of 1 mg/kg docetaxel. The animals were sacrified (n = 5) 2, 6 and 12 hours after injection

The tumors were weighed before treatment. Tumors were digested in the presence of Soluene 350, bleached using 30% hydrogen peroxide and scintillation fluid was added to the solution obtained as described in example 6. The amount of nanocapsules and docetaxel in the tumors was assessed by counting [³H] and [¹⁴C] radioactivity, respectively, using a scintillation counter.

### Evaluation of tumor accumulation at different times

Figure 9 shows the accumulation of Lipid Nanocapsules (LN) and Stealth Lipid Nanocapsules (S-LN) in tumors at different time. Owing to their long circulating and stealth properties, the 3% w/w docetaxel loaded DSPE-PEG₂₀₀₀-6mol% Lipid Nanocapsules in accordance with the invention exhibited higher tumor accumulation (P < 0.05, Student Test) than 3% w/w docetaxel-loaded conventional Lipid Nanocapsules at any time. The amount of S-LN in tumors, was at least 3 times higher than that of conventional LN 12 hours after administration.

Figure 10 represents the amount of Docetaxel accumulated in tumors for Lipid Nanocapsules (LN), Stealth Lipid Nanocapsules (S-LN) and the reference commercial formulation Taxotere^{®} (TXT). Statistically significant differences were calculated by one-way analysis of variance (between formulations at a given time and between the different times for a given formulation). When P value was considered significant (P < 0.05), the post-hoc Scheffé test was used for comparisons among pairs of means. Two hours after intravenous administration, the amounts of docetaxel accumulated in tumors were not significantly different for the three formulations (P = 0.954). Six hours after administration the amount of docetaxel accumulated in tumors was significantly higher for S-LN than TXT. Higher differences could be noted 12 hours after administration: although the amounts of docetaxel between TXT and LN were considered not significantly different, the S-LN allowed more docetaxel to be delivered in tumors than did the LN or TXT (the amount of docetaxel in tumors was however not statistically different between TXT and LN). The analysis of data for each formulation showed that the amount of docetaxel, administered as the TXT formulation, didn't vary with time (P = 0.995). The amount of docetaxel, administered in conventional LN, slightly increased with time in tumors but with no statistical significance (P = 0.069). However for the docetaxel-loaded S-LN in accordance with the invention, the amount of docetaxel in tumors significantly increased with time.

In conclusion, the docetaxel loaded Stealth Lipid Nanocapsules in accordance with the invention were shown to accumulate more in tumors than the commercial solution of docetaxel (Taxotere^{®}), when administered at the same dose. Moreover, the applicant has noted that the drug loaded Stealth Lipid Nanocapsules of the present invention, owing to their long circulating properties, were more effective than conventional Lipid Nanocapsules in terms of amount of anticancer agent targeted at the tumor site.

## Claims

1. Stealth lipid nanocapsules with a diameter of less than 300 nm consisting of an essentially lipid core which is liquid or semi-liquid at ambient temperature, and an outer lipid envelope comprising at least one hydrophilic surfactant which is lipidic in nature, at least one lipophilic surfactant which is lipidic in nature and at least one amphiphilic derivative of poly (ethylene glycol), wherein the molar mass of the poly (ethylene glycol) component is greater than or equal to 1 000 g/mol.

2. The stealth lipid nanocapsules according to Claim 1 wherein the molar mass of the poly (ethylene glycol) component is greater than or equal to 2 000 g/mol.

3. The stealth lipid nanocapsules according to Claim 1 or 2, wherein said lipophilic surfactant is a lecithin, the phosphatidylcholine proportion of which is at least equal to 95%, preferably greater than 99%.

4. The stealth lipid nanocapsules according to any of Claims 1 to 3, wherein said lipophilic surfactant has a gel/liquid transition temperature of at least equal to 25°C, preferably greater than 37°C.

5. The stealth lipid nanocapsules according to any of Claims 1 to 4, wherein the lipophilic surfactant is a phospholipid comprising acyl chains of at least 16 carbon atoms.

6. The stealth lipid nanocapsules according to Claim 5, wherein said lipophilic surfactant is selected from the group consisting of HSPC (hydrogenated soy phosphatidylcholine), DSPC (distearoylphosphatidylcholine) and DPPC (dipalmitoylphosphatidylcholine), and also mixtures thereof.

7. The stealth lipid nanocapsules according to any of Claims 1 to 6, wherein said lipophilic surfactant represents between 5 and 30 mol% of the molecules making up said outer lipid envelope.

8. The stealth lipid nanocapsules according to any of Claims 1 to 7, wherein said hydrophilic surfactant is selected from the group consisting of poly (ethylene glycol) alkyl esters and poly (ethylene glycol) alkyl ethers, and also mixtures thereof.

9. The stealth lipid nanocapsules according to Claim 8, wherein said hydrophilic surfactant is a nonionic surfactant of the poly (ethylene glycol)-660 12- hydroxystearate type comprising a chain of 15 units of ethylene glycol.

10. The stealth lipid nanocapsules according to any of Claims 1 to 9, wherein said hydrophilic surfactant represents between 60 and 90 mol% of the molecules making up said outer lipid envelope, preferably 80mol%.

11. The stealth lipid nanocapsules according to any of Claims 1 to 10, wherein said amphiphilic derivative of poly (ethylene glycol) comprises a hydrophobic component which allows it to be anchored in said outer lipid envelope and a hydrophilic component of the poly (ethylene glycol) type facing the outside of said lipid nanocapsules, conferring hydrophilic properties at the surface thereof.

12. The stealth lipid nanocapsules according to any of Claims 1 to 11, wherein said amphiphilic derivative of poly (ethylene glycol) is chosen from biodegradable phospholipids.

13. The stealth lipid nanocapsules according to Claim 12, wherein said biodegradable phospholipids are selected from the group consisting of
DPPE-PEGx (dipalmitoylphosphatidylethanolamine),
DSPE-PEGx (distearoylphosphatidylethanolamine),
DOPE-PEGx (dioleoylphosphatidylethanolamine), and
POPE-PEGx (palmitoyloleylphosphatidylethanolamine),
in which x is greater than or equal to 1 000 g/mol, and also mixtures thereof.

14. The stealth lipid nanocapsules according to Claim 13, wherein said biodegradable phospholipids are selected from the group consisting of DSPE-PEG₂₀₀₀,DSPE-PEG₃₀₀₀ and DSPE-PEG₅₀₀₀, and also.mixtures thereof.

15. The stealth lipid nanocapsules according to any of Claims 1 to 14, wherein said amphiphilic derivative of poly (ethylene glycol) represents between 0.5 and 12 mol% of the molecules making up said outer lipid envelope, preferably between 1 and 10 mol%.

16. The stealth lipid nanocapsules according to any of Claims 1 to 15, wherein said essentially lipid core represents between 20 and 60% by weight relative to the total weight of said nanocapsules, preferably between 25 and 50% by weight relative to the total weight of said nanocapsules.

17. The stealth lipid nanocapsules according to any of Claims 1 to 16, wherein said essentially lipid core is composed of fatty acid esters and/or of triglycerides and/or of oil, and/or mixtures thereof.

18. The stealth lipid nanocapsules according to Claim 17, wherein the triglycerides making up said essentially lipid core are chosen from the medium chain triglycerides carrying from 6 to 14 carbon atoms, caprylic/capric triglycerides, and mixtures thereof.

19. The stealth lipid nanocapsules according to Claim 17, wherein the fatty acid esters making up said essentially lipid core are selected from the group consisting of medium chain fatty acids carrying from 8 to 18 carbon atoms.

20. The stealth lipid nanocapsules according to Claim 19, wherein the fatty acid esters making up said essentially lipid core are selected from the group consisting of ethyl palmitate, ethyl oleate, ethyl myristate, isopropyl myristate, octyldodecyl myristate, and mixtures thereof.

21. The stealth lipid nanocapsules according to any of Claims 1 to 20, being between 50 and 150 nm, in diameter, preferably between 80 and 120 nm, in diameter.

22. The stealth lipid nanocapsules according to any of Claims 1 to 21, wherein the outer surface of said outer lipid envelope is hydrophilic in nature, and the essentially lipid core is lipophilic in nature.

23. The stealth lipid nanocapsules according to any of Claims 1 to 22, carrying at their surface specific ligands which confer upon them the ability to actively target cells having receptors for these ligands, in particular tumor cells.

24. The stealth lipid nanocapsules according to Claim 23, wherein said ligand is selected from the groups consisting of the saccharide, oligosaccharide, vitamin, oligopeptide, antibody fragment and monoclonal antibody type.

25. The stealth lipid nanocapsules according to any of Claims 1 to 24, having a half-life of at least 2 hours in the blood compartment of the host to which they are administered.

26. The stealth lipid nanocapsules according to any of Claims 1 to 25, being able to rapidly release most of their contents by biodegradation, and in particular by enzymatic digestion.

27. The stealth lipid nanocapsules according to any of Claims 1 to 26, containing one or more active principles.

28. The stealth lipid nanocapsules according to Claim 27, containing one or more anticancer active principles which are mainly lipophilic in nature.

29. The stealth lipid nanocapsules according to Claim 28 wherein the anticancer active principles are selected from the group consisting of paclitaxel and derivatives thereof, such as docetaxel, camptothecin and derivatives thereof, such as irinotecan, topotecan, rubitecan, and busulfan, chlorambucil, phthalocyanins, carotenoids and daunomycin.

30. The stealth lipid nanocapsules according to Claim 27, containing one or more anticancer active principles which are amphiphilic in nature.

31. The stealth lipid nanocapsules according to Claim 30, wherein the anticancer active principles are selected from the group consisting of cytarabine, cyclophosphamide, methotrexate, fluoro derivatives, such as 5-fluorouracil or 5-fluorouridine, and doxorubicin.

32. The stealth lipid nanocapsules according to Claim 27, containing one or more active principles selected from the group consisting of anti-inflammatories, corticoids, antibiotics, analgesics and anti-infectious agents.

33. The stealth lipid nanocapsules according to Claim 32 containing dexamethasone, indomethacin, ibuprofen, ketoprofen, ketoconazole, prostaglandin E1 or amphotericin B.

34. A method for preparing the nanocapsules according to any of Claims 1 to 33, comprising preformating nanocapsules lacking amphiphilic derivative of poly (ethylene glycol), and then post-inserting said amphiphilic derivatives of poly (ethylene glycol) into the surface of these nanocapsules.

35. The method according to Claim 34, wherein said preformation step comprises the synthesis of nanocapsules lacking amphiphilic derivative of poly (ethylene glycol), according to the phase inversion of an oil/water emulsion brought about by several cycles of increase and decrease in temperature.

36. The method according to Claim 34, wherein said post-insertion step comprises a first step of coincubation of the preformed nanocapsules in the presence of the amphiphilic derivative of poly (ethylene glycol), and then a second "quenching" step during which the amphiphilic derivative of poly (ethylene glycol)/preformed nanocapsules mixture thus obtained is abruptly cooled so as to reach a temperature of between 0 and 5°C.

37. The method according to Claim 36, wherein the step of coincubation of the amphiphilic derivative of poly (ethylene glycol)/preformed nanocapsules mixture is carried out at a temperature very slightly higher than the gel/liquid phase transition temperature of said lipophilic surfactant which is lipid in nature, but lower than the phase inversion temperature of the amphiphilic derivative of poly (ethylene glycol)/ preformed nanocapsules mixture.

38. The method according to any of Claims 34 to 37, wherein the diameter of the nanocapsules is adjusted by adjusting the proportion and the length of the hydrophilic chains of the amphiphilic derivative when it is introduced in the post- insertion step.

39. A method for preparing the nanocapsules according to any of Claims 1 to 33, wherein the diameter of the nanocapsules is adjusted by adjusting the proportions of salt and of hydrophilic surfactant, and the purity of the lipophilic surfactant, in the starting mixture of the conventional method of synthesis.

40. The method according to any of Claims 34 to 39, being free of any organic solvent and using only biodegradable compounds approved for parenteral use.

41. The method according to any of Claims 34 to 40, wherein said nanocapsules are sterilized by sterilizing filtration through a filter with a diameter of 0.45 µm to 0.22 µm.

42. The method according to any of Claims 34 to 41, wherein said nanocapsules are lyophilized and then reconstituted extemporaneously in the form of a colloidal suspension.

43. A pharmaceutical composition comprising the lipid nanocapsules according to any of Claims 1 to 33.

44. The pharmaceutical composition according to Claim 43, being in the form of a colloidal aqueous suspension containing said lipid nanocapsules.

45. Use of the lipid nanocapsules according to any of Claims 27 to 31 for the preparation of a medicament for the treatment of cancers, in particular solid or circulating tumors, by intravenous administration.

46. The use according to Claim 45, for the preparation of a medicament for treating circulating or solid tumors by active targeting.

47. The use according to Claim 45, for the preparation of a medicament for treating solid tumors by passive targeting subsequent to the extravasation of said nanocapsules through the tumor capillaries.

48. Use of the lipid nanocapsules according to claim 32 or 33 for the preparation of a medicament for treating inflammations and/or infections of tissues.

49. The use according to any of Claims 45 to 48, wherein the medicinal product is intended to be administered parenterally, or injected into the circulation of a subject intravascularly, in particular intravenously or intra-arterially, intraperitonealy, intramuscularly, subcutaneously or intra-articularly.

50. Use of the nanocapsules according to any of Claims 1 to 26, for the preparation of a medicament for taking up hydrophobic molecules present in the blood circulation subsequent to an instance of poisoning.

51. The use according to any of claims 45 to 50, **characterized in that** the toxicity of the active principle (s) against the healthy tissues is reduced.

## Patentansprüche

1. Stealth-Lipidnanokapseln mit einem Durchmesser von weniger als 300 nm, die aus einem Kern im Wesentlichen aus Lipid, der bei Umgebungstemperatur flüssig oder halbflüssig ist, und einer äußeren Lipidhülle bestehen, welche mindestens ein hydrophiles Tensid, das lipidischer Natur ist, mindestens ein lipophiles Tensid, das lipidischer Natur ist, und mindestens ein amphiphiles Derivat von Poly(ethylenglycol) umfasst, wobei die Molmasse der Poly(ethylenglycol)-Komponente größer als oder gleich 1000 g/Mol ist.

2. Stealth-Lipidnanokapseln nach Anspruch 1, bei denen die Molmasse der Poly(ethylenglycol)-Komponente größer als oder gleich 2000 g/Mol ist.

3. Stealth-Lipidnanokapseln nach Anspruch 1 oder 2, bei dem das lipophile Tensid ein Lecithin ist, dessen Phosphatidylcholin-Teil mindestens gleich 95 %, vorzugsweise größer als 99 % ist.

4. Stealth-Lipidnanokapseln nach irgendeinem der Ansprüche 1 bis 3, bei denen das lipophile Tensid eine Gel-Flüssigkeit-Übergangstemperatur von mindestens 25 °C, vorzugsweise mehr als 37 °C aufweist.

5. Stealth-Lipidnanokapseln nach irgendeinem der Ansprüche 1 bis 4, bei denen das lipophile Tensid ein Phospholipid ist, das Acylketten mit mindestens 16 Kohlenstoffatomen umfasst.

6. Stealth-Lipidnanokapseln nach Anspruch 5, bei denen das lipophile Tensid ausgewählt ist aus der Gruppe bestehend aus HSPC (hydriertem Sojaphosphatidylcholin), DSPC (Distearoylphosphatidylcholin) und DPPC (Dipalmitoylphosphatidylcholin) und auch deren Mischungen.

7. Stealth-Lipidnanokapseln nach irgendeinem der Ansprüche 1 bis 6, bei denen das lipophile Tensid zwischen 5 und 30 Mol% der Moleküle darstellt, welche die äußere Lipidhülle ausmachen.

8. Stealth-Lipidnanokapseln nach irgendeinem der Ansprüche 1 bis 7, bei denen das hydrophile Tensid ausgewählt ist aus der Gruppe bestehend aus Poly(ethlyenglycol)alkylestern und Poly(ethylengycol)alkylethern und auch deren Mischungen.

9. Stealth-Lipidnanokapseln nach Anspruch 8, bei denen das hydrophile Tensid ein nichtionisches Tensid vom Poly(ethylenglycol)-660-12-hydroxystearat-Typ ist, das eine Kette von 15 Einheiten Ethlyenglycol umfasst.

10. Stealth-Lipidnanokapseln nach irgendeinem der Ansprüche 1 bis 9, bei denen das hydrophile Tensid zwischen 60 und 90 Mol% der Moleküle darstellt, welche die äußere Lipidhülle ausmachen, vorzugsweise 80 Mol%.

11. Stealth-Lipidnanokapseln nach irgendeinem der Ansprüche 1 bis 10, bei denen das amphiphile Derivat von Poly(ethylenglycol) eine hydrophobe Komponente, die ermöglicht, dass es in der äußeren Lipidhülle verankert wird, und eine hydrophile Komponente vom Poly(ethlyenglycol)-Typ umfasst, die zur Außenseite der Lipidnanokapseln weist, was an deren Oberfläche hydrophile Eigenschaften bereitstellt.

12. Stealth-Lipidnanokapseln nach irgendeinem der Ansprüche 1 bis 11, bei denen das amphiphile Derivat von Poly(ethylenglycol) aus bioabbaubaren Phospholipiden ausgewählt ist.

13. Stealth-Lipidnanokapseln nach Anspruch 12, bei denen die bioabbaubaren Phospholipide ausgewählt sind aus der Gruppe bestehend aus:
DPPE-PEGx (Dipalmitoylphosphatidylethanolamin),
DSPE-PEGx (Distearoylphosphatidylethanolamin),
DOPE-PEGx (Dioleoylphosphatidylethanolamin) und
POPE-PEGx (Palmitoyloleylphosphatidylethanlolamin),
worin x größer als oder gleich 1000 g/Mol ist, und auch deren Mischungen.

14. Stealth-Lipidnanokapseln nach Anspruch 13, bei denen die bioabbaubaren Phospholipide ausgewählt sind aus der Gruppe bestehend aus DSPE-PEG₂₀₀₀, DSPE-PEG₃₀₀₀ und DSPE-PG₅₀₀₀ und auch deren Mischungen.

15. Stealth-Lipidnanokapseln nach irgendeinem der Ansprüche 1 bis 14, bei denen das amphiphile Derivat von Poly(ethylenglycol) zwischen 0,5 und 12 Mol% der Moleküle darstellt, welche die äußere Lipidhülle ausmachen, vorzugsweise zwischen 1 und 10 Mol%.

16. Stealth-Lipidnanokapseln nach irgendeinem der Ansprüche 1 bis 15, bei denen der Kern aus im Wesentlichen Lipid zwischen 20 und 60 Gew.-%, bezogen auf das Gesamtgewicht der Nanokapseln, vorzugsweise zwischen 25 und 50 Gew.-%, bezogen auf das Gesamtgewicht der Nanokapseln, darstellt.

17. Stealth-Lipidnanokapseln nach irgendeinem der Ansprüche 1 bis 16, bei denen der Kern aus im Wesentlichen Lipid aus Fettsäureestern und/oder Triglyceriden und/oder aus Öl und/oder Mischung derselben zusammengesetzt ist.

18. Stealth-Lipidnanokapseln nach Anspruch 17, bei denen die Triglyceride, die den Kern aus im Wesentlichen Lipid ausmachen, ausgewählt sind aus den mittelkettigen Triglyceriden, die 6 bis 14 Kohlenstoffatome tragen, Capryl/Caprintriglyceriden und deren Mischungen.

19. Stealth-Lipidnanokapseln nach Anspruch 17, bei denen die Fettsäureester, welche den Kern aus im Wesentlichen Lipid ausmachen, ausgewählt sind aus der Gruppe bestehend aus mittelkettigen Fettsäuren, die 8 bis 18 Kohlenstoffatome tragen.

20. Stealth-Lipidnanokapseln nach Anspruch 19, bei denen die Fettsäureester, die den Kern aus im Wesentlichen Lipid ausmachen, ausgewählt sind aus der Gruppe bestehend aus Ethylpalmitat, Ethyloleat, Ethylmyristat, Isopropylmyristat, Octyldodecylmyristat und deren Mischungen.

21. Stealth-Lipidnanokapseln nach irgendeinem der Ansprüche 1 bis 20, die einen Durchmesser zwischen 50 und 150 nm, vorzugsweise einen Durchmesser zwischen 80 und 120 nm aufweisen.

22. Stealth-Lipidnanokapseln nach irgendeinem der Ansprüche 1 bis 21, bei denen die äußere Oberfläche der äußeren Lipidhülle hydrophiler Natur ist und der Kern aus im Wesentlichen Lipid lipophiler Natur ist.

23. Stealth-Lipidnanokapseln nach irgendeinem der Ansprüche 1 bis 22, welche an ihrer Oberfläche spezifische Liganden tragen, welche sie mit der Fähigkeit versehen, aktiv Zellen mit Rezeptoren für diese Liganden, insbesondere Tumorzellen, anzusteuern.

24. Stealth-Lipidnanokapseln nach Anspruch 23, bei denen der Ligand ausgewählt ist aus der Gruppe bestehend aus dem Saccharid-, Oligosaccharid-, Vitamin-, Oligopeptid-, Antikörperfragment- und Monoklonaler-Antikörper-Typ.

25. Stealth-Lipidnanokapseln nach irgendeinem der Ansprüche 1 bis 24 mit einer Halbwertszeit von mindestens 2 Stunden im Blutkompartiment des Wirts, an den sie verabreicht werden.

26. Stealth-Lipidnanokapsein nach irgendeinem der Ansprüche 1 bis 25, die in der Lage sind, das meiste ihres Inhalts durch Bioabbau, insbesondere durch enzymatischen Verdau, schnell freizusetzen.

27. Stealth-Lipidnanokapseln nach irgendeinem der Ansprüche 1 bis 26, die einen oder mehrere Wirkstoffe enthalten.

28. Stealth-Lipidnanokapseln nach Anspruch 27, die einen oder mehrere Antikrebs-Wirkstoffe enthalten, die hauptsächlich lipophiler Natur sind.

29. Stealth-Lipidnanokapseln nach Anspruch 28, bei denen die Antikrebs-Wirkstoffe ausgewählt sind aus der Gruppe bestehend aus Paclitaxel und dessen Derivaten, wie Docetaxel, Camptothecin und dessen Derivaten, wie Irinotecan, Topotecan, Rubitecan und Busulfan, Chlorambucil, Phthalocyaninen, Carotinoiden und Daunomycin.

30. Stealth-Lipidnanokapseln nach Anspruch 27, die einen oder mehrere Antikrebs-Wirkstoffe enthalten, die amphiphiler Natur sind.

31. Stealth-Lipidnanokapseln nach Anspruch 30, bei denen die Antikrebs-Wirkstoffe ausgewählt sind aus der Gruppe bestehend aus Cytarabin, Cyclophosphamid, Methotrexat, Fluorderivaten, wie 5-Fluoruracil oder 5-Fluoruridin, und Doxorubicin.

32. Stealth-Lipidnanokapseln nach Anspruch 27, die einen oder mehrere Wirkstoffe enthalten, die ausgewählt sind aus der Gruppe bestehend aus entzündungshemmenden Stoffen, Corticoiden, Antibiotika, Analgetika und infektionshemmenden Mitteln.

33. Stealth-Lipidnanokapseln nach Anspruch 32, die Dexamethason, Indometacin, Ibuprofen, Ketoprofen, Ketoconazol, Prostaglandin E1 oder Amphotericin B enthalten.

34. Verfahren zum Herstellen von Nanokapseln nach irgendeinem der Ansprüche 1 bis 33, umfassend die Vorbildung von Nanokapseln, denen ein amphiphiles Derivat von Poly(ethylenglycol) fehlt, und dann das nachträgliche Einfügen des amphiphilen Derivats von Poly(ethlyenglycol) in die Oberfläche dieser Nanokapseln.

35. Verfahren nach Anspruch 34, bei dem der Vorformungsschritt die Synthese von Nanokapseln, welchen des amphiphile Derivat von Poly(ethlyenglycol) fehlt, gemäß der Phaseninversion einer Öl/Wasser-Emulsion umfasst, die durch mehrere Zyklen der Erhöhung und Erniedrigung der Temperatur herbeigeführt wird.

36. Verfahren nach Anspruch 34, bei dem der Schritt des nachträglichen Einfügens einen ersten Schritt der Coinkubation der vorgeformten Nanokapseln in Anwesenheit des amphiphilen Derivats von Poly(ethylenglycol) und dann einen zweiten "Quench"-Schritt umfasst, während dessen die so erhaltene Mischung des amphiphilen Derivats von Poly(ethlyenglycol) / vorgeformten Nanokapseln abrupt so abgekühlt wird, dass eine Temperatur zwischen 0 und 5 °C erreicht wird.

37. Verfahren nach Anspruch 36, bei dem der Schritt der Coinkubation der Mischung des amphiphilen Derivats von Poly(ethlyenglycol) / vorgeformten Nanokapseln bei einer Temperatur durchgeführt wird, die ganz wenig höher ist als die Gel/Flüssigkeit-Phasenübergangstemperatur des lipophilen Tensids, das Lipidnatur besitzt, aber niedriger als die Phaseninversionstemperatur der Mischung des amphiphilen Derivats von Poly(ethylenglycol) / vorgeformten Nanokapseln.

38. Verfahren nach irgendeinem der Ansprüche 34 bis 37, bei dem der Durchmesser der Nanokapseln angepasst wird, indem der Anteil und die Länge der hydrophilen Ketten des amphiphilen Derivats angepasst werden, wenn es in dem Schritt des nachträglichen Einfügens eingeführt wird.

39. Verfahren zur Herstellung von Nanokapseln nach irgendeinem der Ansprüche 1 bis 33, bei dem der Durchmesser der Nanokapseln eingestellt wird, indem man die Anteile von Salz und von hydrophilem Tensid und die Reinheit des lipophilen Tensids in der Anfangsmischung des herkömmlichen Syntheseverfahrens einstellt.

40. Verfahren nach einem der Ansprüche 34 bis 39, das frei von jeglichem organischem Lösungsmittel ist und nur bioabbaubare Verbindungen verwendet, die für eine parenterale Verwendung zugelassen sind.

41. Verfahren nach irgendeinem der Ansprüche 34 bis 40, bei dem die Nanokapseln durch sterilisierende Filtration durch ein Filter mit einem Durchmesser von 0,45 µm bis 0,22 µm sterilisiert werden.

42. Verfahren nach irgendeinem der Ansprüche 34 bis 41, bei dem die Nanokapseln lyophilisiert und dann vor Gebrauch in Form einer kolloidalen Suspension rekonstituiert werden.

43. Pharmazeutische Zusammensetzung, umfassend die Lipidnanokapseln nach irgendeinem der Ansprüche 1 bis 33.

44. Pharmazeutische Zusammensetzung nach Anspruch 43, die in Form einer kolloidalen wässrigen Suspension vorliegt, welche die Lipidnanokapseln enthält.

45. Verwendung der Lipidnanokapseln nach irgendeinem der Ansprüche 27 bis 31 für die Herstellung eines Medikaments zur Behandlung von Krebsen, insbesondere festen oder zirkulierenden Tumoren, durch intravenöse Verabreichung.

46. Verwendung nach Anspruch 45 für die Herstellung eines Medikaments zur Behandlung von zirkulierenden oder soliden Tumoren durch aktives Ansteuern.

47. Verwendung nach Anspruch 45 für die Herstellung eines Medikaments zur Behandlung von festen Tumoren durch passives Ansteuern nach der Extravasation der Nanokapseln durch die Tumorkapillaren.

48. Verwendung der Lipidnanokapseln nach Anspruch 32 oder 33 für die Herstellung eines Medikaments zur Behandlung von Entzündungen oder Infektionen von Geweben.

49. Verwendung nach irgendeinem der Ansprüche 45 bis 48, bei der das medizinische Produkt parenteral zu verabreichen ist oder intravaskulär in die Zirkulation eines Patienten zu injizieren ist, insbesondere intravenös oder intrarteriell, intraperitoneal, intramuskulär, subkutan oder intraartikulär.

50. Verwendung der Nanokapseln nach irgendeinem der Ansprüche 1 bis 26 für die Herstellung eines Medikaments zur Aufnahme von hydrophoben Molekülen, die im Blutkreislauf vorliegen, nach einem Vergiftungsvorfall.

51. Verwendung nach irgendeinem der Ansprüche 45 bis 50, **dadurch gekennzeichnet, dass** die Toxizität des Wirkstoffs oder der Wirkstoffe für die gesunden Gewebe verringert ist.

## Revendications

1. Nanocapsules lipidiques furtives d'un diamètre inférieur à 300 nm, constituées d'un noyau essentiellement lipidique qui est liquide ou semi-liquide à température ambiante et d'une enveloppe lipidique externe comprenant au moins un surfactant hydrophile qui est lipidique de nature, au moins un surfactant lipophile qui est lipidique de nature et au moins un dérivé amphiphile de poly(éthylène glycol), où la masse molaire du composant de poly(éthylène glycol) est supérieure ou égale à 1000 g/mol.

2. Nanocapsules lipidiques furtives selon la revendication 1, où la masse molaire du composant de poly(éthylène glycol) est supérieure ou égale à 2000 g/mol.

3. Nanocapsules lipidiques furtives selon la revendication 1 ou 2, dans lesquelles ledit surfactant lipophile est une lécithine, dont la proportion de phosphatidylcholine est au moins égale à 95 %, de préférence supérieure à 99%.

4. Nanocapsules lipidiques furtives selon l'une quelconque des revendications 1 à 3, dans lesquelles ledit surfactant lipophile a une température de transition gel/liquide au moins égale à 25 °C, de préférence supérieure à 37 °C.

5. Nanocapsules lipidiques furtives selon l'une quelconque des revendications 1 à 4, dans lesquelles le surfactant lipophile est un phospholipide comprenant des chaînes acyle d'au moins 16 atomes de carbone.

6. Nanocapsules lipidiques furtives selon la revendication 5, dans lesquelles ledit surfactant lipophile est choisi dans le groupe comprenant la HSPC (phosphatidylcholine hydrogénée de soja), la DSPC (distéaroyl-phosphatidylcholine) et la DPPC (dipalmitoyl-phosphatidylcholine) et également des mélanges de celles-ci.

7. Nanocapsules lipidiques furtives selon l'une quelconque des revendications 1 à 6, dans lesquelles ledit surfactant lipophile représente entre 5 et 30 % en mole des molécules constituant ladite enveloppe lipidique externe.

8. Nanocapsules lipidiques furtives selon l'une quelconque des revendications 1 à 7, dans lesquelles ledit surfactant hydrophile est choisi dans le groupe comprenant des esters alkyliques de poly(éthylène glycol) et des éthers alkyliques de poly(éthylène glycol) et également des mélanges de ceux-ci.

9. Nanocapsules lipidiques furtives selon la revendication 8, dans lesquelles ledit surfactant hydrophile est un surfactant non ionique du type 12-hydroxystéarate de poly(éthylène glycol)-660 comprenant une chaîne de 15 unités d'éthylène glycol.

10. Nanocapsules lipidiques furtives selon l'une quelconque des revendications 1 à 9, dans lesquelles ledit surfactant hydrophile représente entre 60 et 90 % en mole des molécules constituant ladite enveloppe lipidique externe, de préférence 80 % en mole.

11. Nanocapsules lipidiques furtives selon l'une quelconque des revendications 1 à 10, dans lesquelles ledit dérivé amphiphile de poly(éthylène glycol) comprend un composant hydrophobe qui lui permet de s'ancrer dans ladite enveloppe lipidique externe et un composant hydrophile du type poly(éthylène glycol) faisant face à l'extérieur desdites nanocapsules lipidiques, conférant des propriétés hydrophiles à la surface de celles-ci.

12. Nanocapsules lipidiques furtives selon l'une quelconque des revendications 1 à 11, dans lesquelles ledit dérivé amphiphile de poly(éthylène glycol) est choisi parmi des phospholipides biodégradables.

13. Nanocapsules lipidiques furtives selon la revendication 12, dans lesquelles lesdits phospholipides biodégradables sont choisis dans le groupe comprenant
la DPPE-PEGₓ (dipalmitoyl-phosphatidyl-éthanolamine),
la DSPE-PEGₓ (distéaroyl-phosphatidyl-éthanolamine),
la DOPE-PEGₓ (dioléoyl-phosphatidyl-éthanolamine), et
la POPE-PEGₓ (palmitoyloléyl-phosphatidyl-éthanolamine),
dans lesquelles x est supérieur ou égal à 1000 g/mol et également des mélanges de celles-ci.

14. Nanocapsules lipidiques furtives selon la revendication 13, dans lesquelles lesdits phospholipides biodégradables sont choisis dans le groupe comprenant la DSPE-PEG₂₀₀₀, la DSPE-PEG₃₀₀₀ et la DSPE-PEG₅₀₀₀ et également des mélanges de celles-ci.

15. Nanocapsules lipidiques furtives selon l'une quelconque des revendications 1 à 14, dans lesquelles ledit dérivé amphiphile de poly(éthylène glycol) représente entre 0,5 et 12 % en mole des molécules constituant ladite enveloppe lipidique externe, de préférence entre 1 et 10 % en mole.

16. Nanocapsules lipidiques furtives selon l'une quelconque des revendications 1 à 15, dans lesquelles ledit noyau essentiellement lipidique représente entre 20 et 60 % en poids par rapport au poids total desdites nanocapsules, de préférence entre 25 et 50 % en poids par rapport au poids total desdites nanocapsules.

17. Nanocapsules lipidiques furtives selon l'une quelconque des revendications 1 à 16, dans lesquelles ledit noyau essentiellement lipidique est composé d'esters d'acides gras et/ou de triglycérides et/ou d'huile et/ou de mélanges de ceux-ci.

18. Nanocapsules lipidiques furtives selon la revendication 17, dans lesquelles les triglycérides constituant ledit noyau essentiellement lipidique sont choisis parmi les triglycérides à chaîne moyenne portant de 6 à 14 atomes de carbone, les triglycérides capryliques/capriques, et des mélanges de ceux-ci.

19. Nanocapsules lipidiques furtives selon la revendication 17, dans lesquelles les esters d'acides gras constituant ledit noyau essentiellement lipidique sont choisis dans le groupe comprenant des acides gras à chaîne moyenne portant de 8 à 18 atomes de carbone.

20. Nanocapsules lipidiques furtives selon la revendication 19, dans lesquelles les esters d'acides gras constituant ledit noyau essentiellement lipidique sont choisis dans le groupe comprenant le palmitate d'éthyle, l'oléate d'éthyle, le myristate d'éthyle, le myristate d'isopropyle, le myristate d'octyldodécyle, et des mélanges de ceux-ci.

21. Nanocapsules lipidiques furtives selon l'une quelconque des revendications 1 à 20, ayant un diamètre entre 50 et 150 nm, de préférence un diamètre entre 80 et 120 nm.

22. Nanocapsules lipidiques furtives selon l'une quelconque des revendications 1 à 21, dans lesquelles la surface externe de ladite enveloppe lipidique externe est hydrophile de nature et le noyau essentiellement lipidique est lipophile de nature.

23. Nanocapsules lipidiques furtives selon l'une quelconque des revendications 1 à 22, portant à leur surface des ligands spécifiques qui leur confèrent la capacité à cibler activement des cellules ayant des récepteurs pour ces ligands, en particulier des cellules tumorales.

24. Nanocapsules lipidiques furtives selon la revendication 23, où ledit ligand est choisi dans les groupes comprenant le type saccharide, oligosaccharide, vitamine, oligopeptide, fragment d'anticorps et anticorps monoclonal.

25. Nanocapsules lipidiques furtives selon l'une quelconque des revendications 1 à 24, ayant une demi-vie d'au moins 2 heures dans le compartiment sanguin de l'hôte auquel elles sont administrées.

26. Nanocapsules lipidiques furtives selon l'une quelconque des revendications 1 à 25, étant capables de libérer rapidement la majeure partie de leur contenu par biodégradation, et en particulier par digestion enzymatique.

27. Nanocapsules lipidiques furtives selon l'une quelconque des revendications 1 à 26, contenant un ou plusieurs principes actifs.

28. Nanocapsules lipidiques furtives selon la revendication 27, contenant un ou plusieurs principes actifs anticancéreux qui sont principalement lipophiles de nature.

29. Nanocapsules lipidiques furtives selon la revendication 28, dans lesquelles les principes actifs anticancéreux sont choisis dans le groupe comprenant le paclitaxel et des dérivés de celui-ci tels que le docétaxel, la camptothécine et des dérivés de celle-ci, tels que l'irinotécan, le topotécan, le rubitécan et le busulfan, le chlorambucil, les phtalocyanines, les caroténoïdes et la daunomycine.

30. Nanocapsules lipidiques furtives selon la revendication 27, contenant un ou plusieurs principes actifs anticancéreux qui sont amphiphiles de nature.

31. Nanocapsules lipidiques furtives selon la revendication 30, dans lesquelles les principes actifs anticancéreux sont choisis dans le groupe comprenant la cytarabine, le cyclophosphamide, le méthotrexate, des dérivés fluorés, tels que le 5-fluorouracile ou la 5-fluorouridine, et la doxorubicine.

32. Nanocapsules lipidiques furtives selon la revendication 27, contenant un ou plusieurs principes actifs choisis dans le groupe comprenant des anti-inflammatoires, des corticoïdes, des antibiotiques, des antalgésiques et des agents anti-infectieux.

33. Nanocapsules lipidiques furtives selon la revendication 32, contenant de la dexaméthasone, de l'indométacine, de l'ibuprofène, du kétoprofène, du kétoconazole, de la prostaglandine E1 ou de l'amphotéricine B.

34. Procédé de préparation des nanocapsules selon l'une quelconque des revendications 1 à 33, comprenant la préformation de nanocapsules manquant du dérivé amphiphile de poly(éthylène glycol) et ensuite l'insertion postérieure desdits dérivés amphiphiles de poly(éthylène glycol) dans la surface de ces nanocapsules.

35. Procédé selon la revendication 34, dans lequel ladite étape de préformation comprend la synthèse de nanocapsules manquant du dérivé amphiphile de poly(éthylène glycol), selon l'inversion de phase d'une émulsion huile/eau provoquée par plusieurs cycles d'augmentation et de diminution de la température.

36. Procédé selon la revendication 34, dans lequel ladite étape d'insertion postérieure comprend une première étape de coincubation des nanocapsules préformées en présence du dérivé amphiphile de poly(éthylène glycol) et ensuite une seconde étape « d'extinction » durant laquelle le mélange dérivé amphiphile de poly(éthylène glycol)/nanocapsules préformées ainsi obtenu est refroidi de manière abrupte de manière à atteindre une température entre 0 et 5 °C.

37. Procédé selon la revendication 36, dans lequel l'étape de coincubation du mélange dérivé amphiphile de poly(éthylène glycol)/nanocapsules préformées est réalisée à une température très légèrement supérieure à la température de transition de phase gel/liquide dudit surfactant lipophile qui est lipidique de nature, mais inférieure à la température d'inversion de phase du mélange dérivé amphiphile de poly(éthylène glycol)/ nanocapsules préformées.

38. Procédé selon l'une quelconque des revendications 34 à 37, dans lequel le diamètre des nanocapsules est ajusté en ajustant la proportion et la longueur des chaînes hydrophiles du dérivé amphiphile lorsqu'il est introduit dans l'étape d'insertion postérieure.

39. Procédé de préparation des nanocapsules selon l'une quelconque des revendications 1 à 33, dans lequel le diamètre des nanocapsules est ajusté en ajustant les proportions de sel et de surfactant hydrophile et la pureté du surfactant lipophile, dans le mélange de départ du procédé traditionnel de synthèse.

40. Procédé selon l'une quelconque des revendications 34 à 39, étant dépourvu de tout solvant organique et utilisant uniquement des composés biodégradables approuvés pour une utilisation parentérale.

41. Procédé selon l'une quelconque des revendications 34 à 40, dans lequel lesdites nanocapsules sont stérilisées par filtration stérilisante à travers un filtre d'un diamètre de 0,45 µm à 0,22 µm.

42. Procédé selon l'une quelconque des revendications 34 à 41, dans lequel lesdites nanocapsules sont lyophilisées et ensuite reconstituées extemporanément sous la forme d'une suspension colloïdale.

43. Composition pharmaceutique comprenant les nanocapsules lipidiques selon l'une quelconque des revendications 1 à 33.

44. Composition pharmaceutique selon la revendication 43, étant sous la forme d'une suspension aqueuse colloïdale contenant lesdites nanocapsules lipidiques.

45. Utilisation des nanocapsules lipidiques selon l'une quelconque des revendications 27 à 31, pour la préparation d'un médicament destiné au traitement de cancers, en particulier des tumeurs solides ou circulantes, par une administration intraveineuse.

46. Utilisation selon la revendication 45, pour la préparation d'un médicament destiné au traitement de tumeurs circulantes ou solides par un ciblage actif.

47. Utilisation selon la revendication 45, pour la préparation d'un médicament destiné au traitement de tumeurs solides par un ciblage passif faisant suite à l'extravasation desdites nanocapsules à travers les capillaires tumoraux.

48. Utilisation des nanocapsules lipidiques selon la revendication 32 ou 33, pour la préparation d'un médicament destiné au traitement d'inflammations et/ou d'infections de tissus.

49. Utilisation selon l'une quelconque des revendications 45 à 48, où le produit médicinal est prévu pour être administré par voie parentérale ou injecté dans la circulation d'un sujet par voie intravasculaire, en particulier par voie intraveineuse ou intra-artérielle, intrapéritonéale, intramusculaire, sous-cutanée ou intra-articulaire.

50. Utilisation des nanocapsules selon l'une quelconque des revendications 1 à 26, pour la préparation d'un médicament destiné à absorber des molécules hydrophobes présentes dans la circulation sanguine à la suite d'un cas d'empoisonnement.

51. Utilisation selon l'une quelconque des revendications 45 à 50, **caractérisée en ce que** la toxicité du/des principe(s) actif(s) contre les tissus sains est réduite.
